# EUROPEAN PATENT APPLICATION

(11) **EP 2 141 166 A1**
(43) Date of publication of application: **06.01.2010**
(21) Application number: 08011782.3
(22) Date of filing: 30.06.2008
(51) Int. Cl.: C07D 473/34, A61K 31/52, A61P 25/00, A61P 29/00

(54) **Adenine receptor ligands**

(71) Applicant: Rheinische Friedrich-Wilhelms-Universität Bonn, 53113 Bonn (DE)
(72) Inventor: Müller, Christa Prof. Dr., 53113 Bonn (DE); Bormann, Thomas, 53123 Bonn (DE); Abdelrahman, Aliaa, 53121 Bonn (DE)
(74) Representative: Bülle, Jan

(57) **Abstract**

The invention relates to adenine receptor ligands useful for treating, alleviating and/or preventing diseases and disorders related to adenine receptor function as well as pharmaceutical compositions comprising such compounds and methods for preparing such compounds. The invention is further directed to the use of these compounds, alone or in combination with other therapeutic agents, for the alleviating, preventing and/or treating diseases and disorders, especially the use as antinociceptive or neuroprotective drugs.

## Description

The invention relates to adenine receptor ligands useful for treating, alleviating and/or preventing diseases and disorders related to adenine receptor function as well as pharmaceutical compositions comprising such compounds and methods for preparing such compounds. The invention is further directed to the use of these compounds, alone or in combination with other therapeutic agents, for alleviating, preventing and/or treating diseases and disorders, especially the use as antinociceptive or neuroprotective drugs.

The adenine receptors belong to a recently discovered family of purinergic receptors, referred to as the P0 receptors (Brunschweiger, A. et al., Curr. Med. Chem., 2006, 13, 289). An orphan G protein-coupled receptor was identified in rats as the adenine receptor with the highest mRNA expression in the small neurons of the dorsal root ganglia (Bender, E. et al., Proc. Natal. Acad. Sci. USA, 2002, 99, 8573). It was shown that the rat adenine receptor is functionally expressed and Gᵢ protein-coupled to adenylate cyclase in native cells and tissues, exhibiting a single high-affinity binding site for adenine at rat brain cortical membranes (Gorzalka, S. et al., Mo/. Pharmacol., 2005, 67, 955). In accordance with the mRNA expression profile, a pronociceptive role of adenine in nociceptive sensory transmission was observed in rats (Matthews, E. A. et al., Neurosci. Lett., 2004, 356, 211). Neuroprotective effects of adenine on Purkinje cell survival were demonstrated to be mediated via a specific adenine binding site in rat brain (Watanabe, S. et al., J. Neurosci. Res., 2003, 74, 754; Watanabe, S. et al., J. Biochem., 2005, 137, 323).

A second murine adenine receptor was identified and functionally characterized from mouse brain, exhibiting an analogous binding profile of selected ligands as the rat adenine receptor (von Kügelgen, I. et al., Mol. Pharmacol., 2008, 73, 469).

Moreover, it was shown, that adenine receptors play a physiological role on sodium reabsorption in the proximal tubule of pig kidneys (Wengert, M. et al., Arch. Biochem. Biophys., 2007, 467, 261).

Radioligand binding studies at membranes of human astrocytoma 1321N1 cells revealed that a human ortholog of the rat adenine receptor exists (Gorzalka, S. et al., Mol. Pharmacol., 2005, 67, 955). The human adenine receptor was also detected in Jurkat T cells and HEK293 cells, which is in accordance with the expression of adenine receptors in porcine kidneys. In HEK293 cells, the adenine receptor is expressed in very high density (Bₘₐₓ value of 8.18 pmol/mg protein) with a single high-affinity binding site (Gorzalka, S. Neuartige G-Protein-gekoppelte Purinrezeptoren: Funktionelle Charakterisierung nativer Adeninrezeptoren und Evaluation neuer Purinrezeptor-Liganden. Dissertation, University of Bonn, 2006). It has been shown that the human adenine receptors are functionally acitve. Besides coupling to inhibition of adenylate cyclase, adenine receptors can also be coupled to activation of adenylate cyclase (Gorzalka, Dissertation, University of Bonn, 2006).

Because the human adenine receptor represents a fundamentally new drug target, the development of potent adenine receptor ligands represents a basic need for the design of novel drugs, targeting the adenine receptor and thus being used for antinociception or neuroprotection or other diseases.

Previous studies showed that even minor modifications of the natural ligand adenine lead to compounds with dramatically diminished receptor affinity. For example, 1-, 3-, 6-, 7-, or 9-methyl-adenine exhibited Kᵢ values in the micromolar range at the rat adenine receptor, whereas adenine itself showed a Kᵢ value of 29.9 nM (Gorzalka, S. et al., Mol. Pharmacol., 2005, 67, 955).

It was an object of the invention to provide compounds that have advantages compared to the compounds of the prior art. The compounds should act as potent and selective adenine receptor ligands, exhibit high water solubility and bioavailability, and thus may be useful as antinociceptive or neuroprotective drugs or for the prevention or treatment of other diseases. Moreover, it was an object of the invention to provide methods for preparing said compounds. It was furthermore an object of the invention to provide compounds and pharmaceutical formulations for the treatment, alleviation and/or prevention of a host of diseases and disorders connected to adenine receptorfunction. It was a further object of the invention to provide the use of these compounds for alleviating, preventing and/or treating diseases and disorders connected to adenine receptorfunction, particularly for, but not limited to the use as antinociceptive or neuroprotective agents.

This object has been solved by the subject-matter of the patent claims.

The present invention is directed to certain adenine derivatives which act as adenine receptor ligands and therefore are useful as antinociceptive or neuroprotective drugs.

In a first aspect, the invention is directed to a compound according to general formula (I): 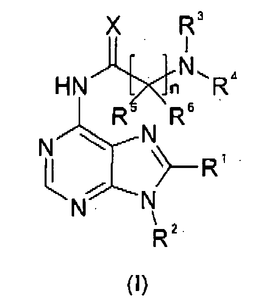 wherein
n is an integer of from 2 to 10; preferably 2, 3, 4 or 5; more preferably 2 or 3;
X is =O, =S, =NH or =NR; preferably =O;
R¹ is -H, -R, -OR, -SR or -NRR'; preferably -H;
R², R³, and R⁴ are independently selected from the group consisting of -H; unsubstituted or substituted, unsaturated or saturated, linear or branched -alkyl; preferably unsubstituted or substituted -C₁-C₁₀ alkyl, -C₁-C₁₀ alkenyl or -C₁-C₁₀ alkynyl; unsubstituted or substituted, unsaturated or saturated -cycloalkyl; preferably unsubstituted or substituted -C₃-C₆ cycloalkyl; unsubstituted or substituted, unsaturated or saturated -heterocycloalkyl; preferably a 5- to 10-membered, unsubstituted or substituted heterocyclic ring wherein 1 to 3 ring atoms are independently selected from N, O and S; unsubstituted or substituted -aryl; preferably -C₆-C₁₄ aryl; unsubstituted or substituted - heteroaryl; preferably a 5- to 10-membered heteroaryl wherein 1 to 4 ring atoms are independently selected from N, O and S; unsubstituted or substituted, unsaturated or saturated -cycloalkyl bonded via unsubstituted or substituted, unsaturated or saturated, linear or branched -alkyl-; unsubstituted or substituted, unsaturated or saturated - heterocycloalkyl bonded via unsubstituted or substituted, unsaturated or saturated, linear or branched -alkyl-; unsubstituted or substituted -aryl bonded via unsubstituted or substituted, unsaturated or saturated, linear or branched -alkyl-; preferably -benzyl; unsubstituted or substituted -heteroaryl.bonded via unsubstituted or substituted, unsaturated or saturated, linear or branched -alkyl-: -C(O)R, -C(O)OR; -C(O)NRR'; and -NRR';
R⁵ and R⁶ are independently selected from the group consisting of -H; -halogen; preferably -F, -Cl, -Br or -I; unsubstituted or substituted, unsaturated or saturated, linear or branched -alkyl; preferably unsubstituted or substituted -C₁-C₁₀ alkyl, -C₁-C₁₀ alkenyl or -C₁-C₁₀ alkynyl; unsubstituted or substituted, unsaturated or saturated -cycloalkyl; preferably unsubstituted or substituted -C₃-C₈ cycloalkyl; unsubstituted or substituted, unsaturated or saturated -heterocycloalkyl; preferably a 5- to 10membered, unsubstituted or substituted heteroalicyclic ring wherein 1 to 3 ring atoms are independently selected from N, O and S; unsubstituted or substituted -aryl; preferably -C₆-C₁₄ aryl; unsubstituted or substituted -heteroaryl; preferably a 5- to 10membered heteroaryl wherein 1 to 4 ring atoms are independently selected from N, O and S; unsubstituted or substituted -aryl bonded via unsubstituted or substituted, unsaturated or saturated, linear or branched -alkyl-; preferably -benzyl; unsubstituted or substituted -heteroaryl bonded via unsubstituted or substituted, unsaturated or saturated, linear or branched -alkyl-; unsubstituted or substituted, unsaturated or saturated 7 to 12-membered bicyclic cycloalkyl ring or heterocycloalkyl ring wherein 1 to 3 ring members are independently selected from N, O and S; an unsubstituted or substituted, unsaturated or saturated 10 to 16-membered tricyclic cycloalkyl ring or heterocycloalkyl ring wherein 1 to 3 ring members are independently selected from N, O and S; unsubstituted or substituted -O-alkyl; preferably -O-C₁-C₁₀ alkyl; more preferably unsubstituted or substituted -C₁-C₄ alkoxy; unsubstituted or substituted -Ocycloalkyl; preferably -O-C₃-C₈ cycloalkyl; -OR; -NRR'; -NO₂: -C(O)R; -C(O)NRR'; -s(O)₁₋₂R; -S(O)₁₋₂OR; and -S(O)₁₋₂NRR';
and
R and R' are independently selected from the group consisting of -H; unsubstituted or substituted, unsaturated or saturated, linear or branched -alkyl; unsubstituted or substituted, unsaturated or saturated -cycloalkyl; unsubstituted or substituted, unsaturated or saturated -heterocycloalkyl; unsubstituted or substituted -aryl;
unsubstituted or substituted -heteroaryl; unsubstituted or substituted -aryl bonded via unsubstituted or substituted, unsaturated or saturated, linear or branched -alkyl-;
unsubstituted or substituted -heteroaryl bonded via unsubstituted or substituted, unsaturated or saturated, linear or branched -alkyl-;
and the physiologically acceptable salts and/or solvates thereof.

When the definition of a particular residue appears several times in the same molecule (e.g. the residue R when R¹ = -R and R⁴ = -C(O)R), the respective residue may have identical or different meanings.

It has been surprisingly found that the compounds according to general formula (I) are useful as mediators (e.g. agonists or antagonists) of adenine receptors and thus, are useful as medicaments.

In general formula (I) integer n can be 2, 3, 4, 5, 6, 7, 8, 9 or 10, The individual residues R⁵ and R⁶ that are bonded to the carbon atoms of the chain -[CR⁵R⁶]ₙ- may be identical or different. In a preferred embodiment of general formula (I), the moiety -[CR⁵R⁶]ₙ- is replaced by the moiety -[CR⁵R⁶']ₚ-[CR⁵''R⁶'']_{q}-[CR⁵'''R⁶''']_{q}- where p+q+r = n and R⁵', R⁵'' and R⁵''' are independently defined as R⁵ above and R⁶', R⁶'' and R⁶''', are independently defined as R⁶ above.

In a preferred embodiment of general formula (I) X is =O, =S or =NH;
R², R³, and R⁴ are independently selected from the group consisting of -H; unsubstituted or substituted -C₁-C₁₀ alkyl; unsubstituted or substituted -C₁-C₁₀ alkenyl; unsubstituted or substituted -C₁-C₁₀ alkynyl; unsubstituted or substituted -C₃-C₆ cycloalkyl; unsubstituted or substituted 5- to 10-membered heterocycloalkyl wherein 1 to 3 ring atoms are independently selected from N, O or S; unsubstituted or substituted -C₆-C₁₄ aryl; unsubstituted or substituted 5- to 10-membered -heteroaryl wherein 1 to 4 ring atoms are independently selected from N, O or S; unsubstituted or substituted -C₁C₁₀ alkyl-C₆-C₁₄ aryl; -C(O)R; -C(O)OR; -C(O)NRR'; and -NRR';
R⁵ and R⁶ are independently selected from the group consisting of -H; -halogen; unsubstituted or substituted -C₁-C₁₀ alkyl; unsubstituted or substituted -C₁-C₁₀ alkenyl; unsubstituted or substituted -C₁-C₁₀ alkynyl; unsubstituted or substituted -C₃-C₈ cycloalkyl; unsubstituted or substituted 5- to 10-membered heterocycloalkyl wherein 1 to 3 ring atoms are independently selected from N, O or S; unsubstituted or substituted -O-C₁-C₁₀ alkyl; unsubstituted or substituted -O-C₃-C₆ cycloalkyl; unsubstituted or substituted C₆-C₁₄ aryl; unsubstituted or substituted 5- to 10-membered -heteroaryl wherein 1 to 4 ring atoms are independently selected from N, O or S; unsubstituted or substituted -C₁-C₁₀ alkyl-C₆-C₁₄ aryl; unsubstituted or substituted -C₁-C₁₀ alkyl-heteroaryl wherein 1 to 4 ring atoms are independently selected from N, O or S; an unsubstituted or substituted, unsaturated or saturated 7 to 12-membered bicyclic cycloalkyl ring or heterocycloalkyl ring wherein 1 to 3 ring members are independently selected from N, O and S; an unsubstituted or substituted, unsaturated or saturated 10 to 16membered tricyclic cycloalkyl ring or heterocycloalkyl ring wherein 1 to 3 ring members are independently selected from N, O and S; -OR; -NRR'; -NO₂; -C(O)R; -C(O)NRR'; -S(O)₂R; -S(O)₂OR; and -S(O)₂NRR';
and
R and R' are independently selected from the group consisting of -H; unsubstituted or substituted -C₁-C₁₀ alkyl, and -C₁-C₁₀ alkyl-C₆-C₁₁ aryl.

In a preferred embodiment of general formula (I),
X is =O; and/or
R¹ is -H; and/or
R² is -H; and/or
R⁵ is -H; and/or
R⁶ is -H.

Preferably, R³ is -H; unsubstituted or substituted -C₁-C₁₀-alkyl; or unsubstituted or substituted -C₁-C₁₀ alkyl-C₆-C₁₁ aryl.

In a preferred embodiment according to the invention, the compound of general formula (I) is represented by general formula (I-A) or (I-B) 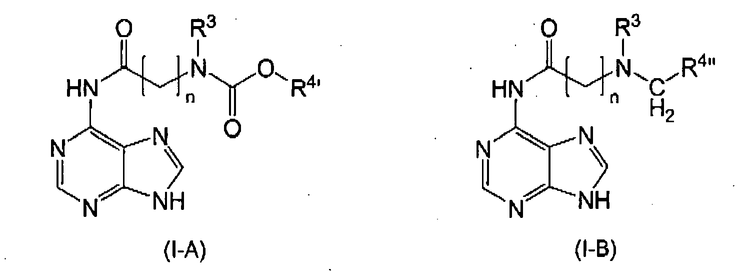 wherein
n and R³ are defined as in any of the preceding claims; and
wherein
R⁴' is unsubstituted or substituted -C₁-C₁₀ alkyl; and
R⁴'' is -H; unsubstituted or substituted -C₁-C₉ alkyl; unsubstituted or substituted -C₆-C₁₄ aryl; or unsubstituted or substituted -C₁-C₉ alkyl-C₆-C₁₄ aryl.

In a preferred embodiment of the compounds according to general formula (I) R³ and R⁴ are both ≠ -H. In another embodiment of the compounds according to general formula (I) R³ is -H and R⁴ is ≠ -H. In still another preferred embodiment of the compounds according to general formula (I) R³ and R⁴ are both = -H.

In a preferred embodiment of the compounds according to general formula (I)
- n: is 2 or 3; and/or
- X: is =O; and/or
- R¹: is -H; and/or
- R²: is -H; and/or
- R³: is selected from the group consisting of -H, -CH₃, -(CH₂)₁₋₅CH₃, and -(CH)₁₋₂C₆H₅; and/or
- R⁴: is selected from the group consisting of -H, -CH₃, -(CH₂)₁₋₂CH₃ and -C(O)OC(CH₃)₃; and/or
- R⁵: is -H; and/or
- R⁶: is -H.

Representative compounds of the invention are shown in Table 1 here below:

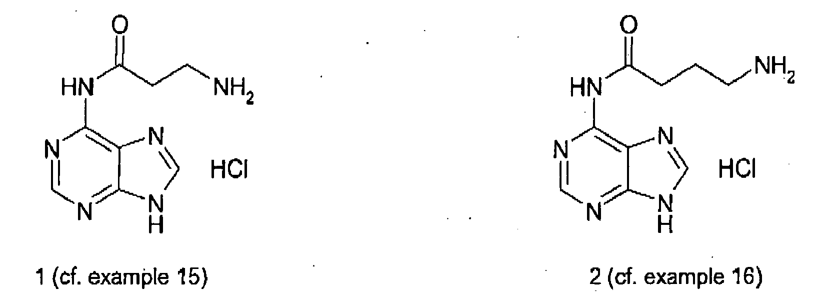

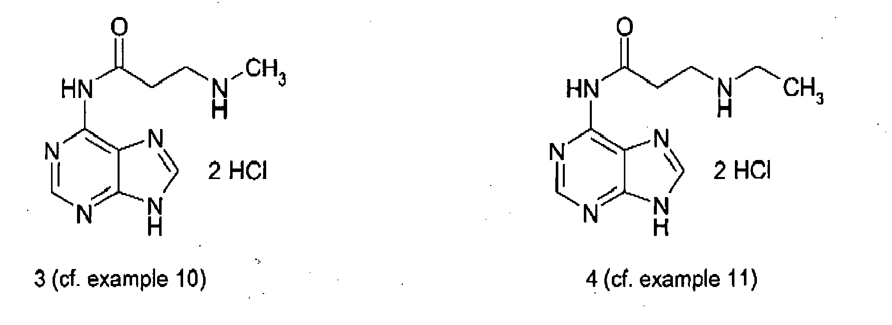

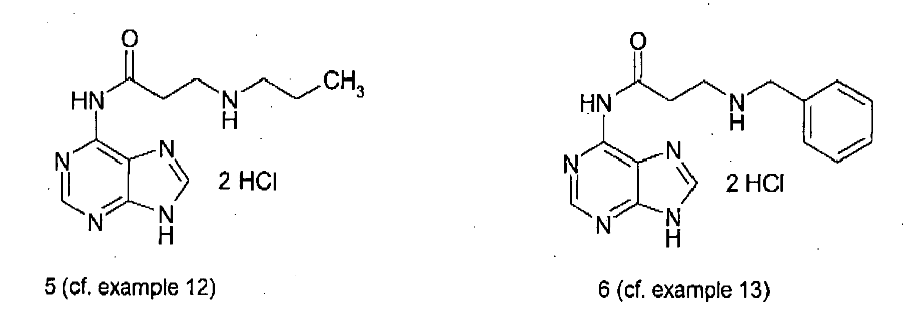

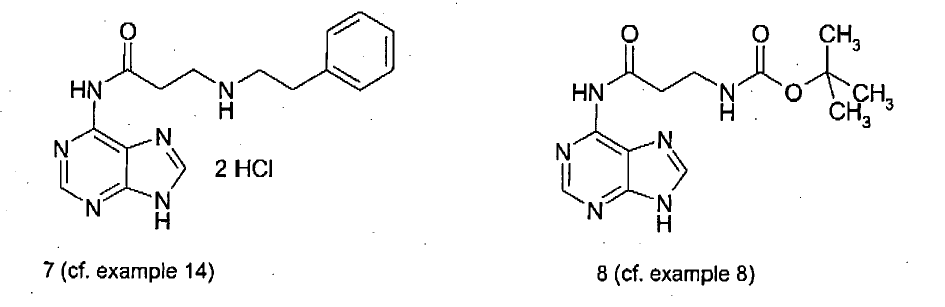

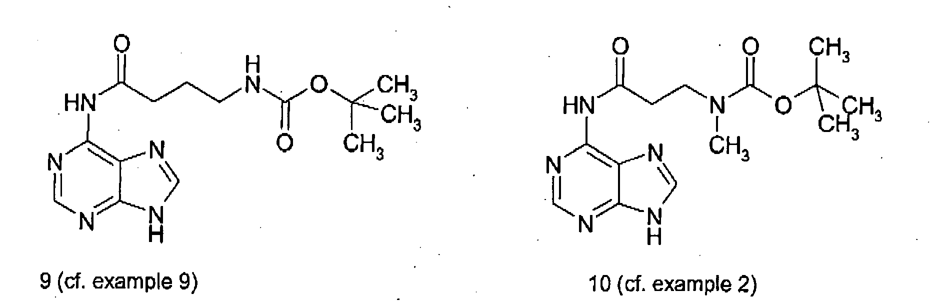

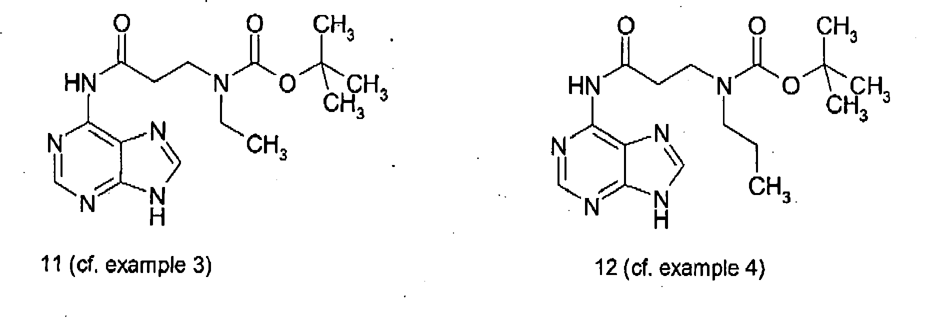

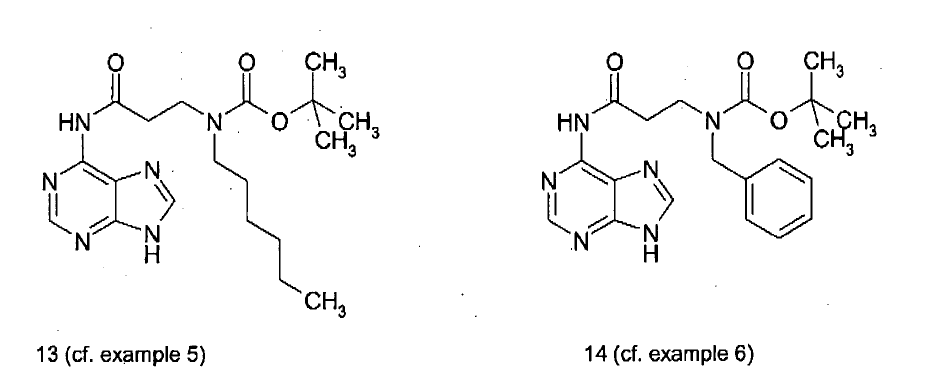

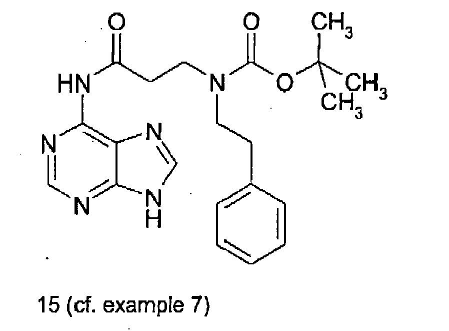

Thus, preferably, the compound of general formula (I) is selected from the group consisting of
- 3-(tert-butyloxycarbonyl-methyl-amino)-N(9H-purin-6-yl)propionamide;
- 3-(tert-butyloxycarbonyl-ethyl-amino)-N(9H-purin-6-yl)propionamide;
- 3-(tert-butyloxycarbonyl-propyl-amino)-N(9H-purin-6-yl)propionamide;
- 3-(tert-butyloxycarbonyl-hexyl-amino)-N(9H-purin-6-yl)propionamide;
- 3-(tert-butyloxycarbonyl-benzyl-amino)-N(9H-purin-6-yl)propionamide;
- 3-(tert-butyloxycarbonyl-phenethyl-amino)-N(9H-purin-6-yl)propionamide;
- 3-tert-butyloxycarbonylamino-N(9H-purin-6-yl)propionamide;
- 4-tert-butyloxylarbonylamino-N(9H-purin-6-yl)butyramide;
- 3-methyl-amino-N(9H-purin-6-yl)propionamide;
- 3-ethyl-amino-N(9H-purin-6-yl)propionamide;
- 3-propyl-amino-N(9H-purin-6-yl)propionamide;
- 3-benzyl-amino-N(9H-purin-6-yl)propionamide;
- 3-phenethyl-amino-N(9H-purin-6-yl)propionamide;
- 3-amino-N(9H-purin-6-yl)propionamide; and
- 4-amino-N(9H-purin-6-yl)butyramide;
and the physiologically acceptable salts and/or solvates thereof.

As used herein, the terms "pharmaceutically acceptable salt" and "physiologiaclly accpetable salt" refer to those salts which retain the biological effectiveness and properties of the compound according to general formula (I). Such salts include, but are not restricted to: (1) an acid addition salt which is obtained by reaction of the free base of the compound according to general formula (I) with inorganic acids such as hydrochloric acid, hydrobromic acid, nitric acid, phosphoric acid, sulfuric acid, and perchloric acid and the like, or with organic acids such as acetic acid, oxalic acid, (D) or (L) malic acid, maleic acid, methanesulfonic acid, ethanesulfonic acid, p-toluenesulfonic acid, salicylic acid, tartaric acid, citric acid, succinic acid or malonic acid and the like, preferably hydrochloric acid or (L)-malic acid; or (2) salts formed when an acidic proton present in the compound according to general formula (I) either is replaced by a metal ion, e. g., an alkali metal ion, such as sodium or potassium, an alkaline earth ion, such as magnesium or calcium, or an aluminum ion; or coordinates with an organic base such as ethanolamine, diethanolamine, triethanolamine, tromethamine, N-methylglucamine, and the like.

In a preferred embodiment, the compounds according to general formula (I) exhibit a Kᵢ value against the rat adenine receptor, preferably under the experimental conditions specified in the experimental section, of at most 100 µM, more preferably at most 10 µM, still more preferably at most 1.0 µM, most preferably at most 0.5 µM and in particular at most 0.1 µM.

In another preferred embodiment, the compounds according to general formula (I) exhibit a Kᵢ value against the human adenine receptor, preferably under the experimental conditions specified in the experimental section, of at most 100 µM, more preferably at most 10 µM, still more preferably at most 1.0 µM, most preferably at most 0.5 µM and in particular at most 0.1 µM.

The compound of general formula (I) may also act as a prodrug. A "prodrug" preferably refers to an agent which is converted into the parent drug *in vivo.* Prodrugs are often useful because, in some situations, they may be easier to administer than the parent drug. They may, for instance, be bioavailable by oral administration whereas the parent drug is not. The prodrug may also have improved solubility in pharmaceutical compositions over the parent drug. An example, without limitation, of a prodrug would be a compound of the present invention which is administered as an ester (the "prodrug") to facilitate transmittal across a cell membrane where water solubility is detrimental to mobility but then is metabolically hydrolyzed to the carboxylic acid, the active entity, once inside the cell where water solubility is beneficial. A prodrug may be converted into the parent drug by various mechanisms, including enzymatic processes and metabolic hydrolysis.

A further example of a prodrug might be a short polypeptide, for example, without limitation, a 2-10 amino acid polypeptide, bonded through a terminal amino group to a carboxy group of a compound of this invention wherein the polypeptide is hydrolyzed or metabolized in vivo to release the active molecule. The prodrugs of compounds of general formula (I) are within the scope of this invention.

Additionally, it is contemplated that compounds of general formula (I) would be metabolized by enzymes in the body of the organism such as a human being to generate a metabolite that can modulate the activity of the adenine receptor. Such metabolites are within the scope of the present invention.

In a preferred embodiment according to the invention, the compound according to general formula (I) is radioactively labeled. Preferred radioactive nuclids include, e.g. ¹¹C, ¹³N, ¹⁵O and ¹⁸F. Radioactively labeled compounds of this type are useful, e.g., as positron emission tracer. Positron emission tomography as a diagnostic tool is known to the skilled person. In this regard it can be referred e.g. to P.E. Valk et al., Positron Emission Tomography; Clinical Practice, Springer; 1 edition, 2006. The scope of the invention also encompasses the use of said radioactively labelled compounds according to general formula (I) as positron emission tracers.

Also encompassed by the present invention are pharmaceutically acceptable free bases, salts or prodrugs of the above compounds of general formula (I) and those set forth in Table 1.

Preferably, unless otherwise stated, the following terms used in the specification and claims have the following meanings:
Unless expressly stated otherwise, "alkyl" preferably refers to an aliphatic hydrocarbon including straight chain, or branched chain groups. Preferably, the alkyl group has 1 to 10 carbon atoms (C₁-C₁₀ alkyl), more preferably 1 to 6 carbon atoms (C₁-C₆ alkyl) and most preferably 1 to 4 carbon atoms (C₁-C₄ alkyl), e. g., methyl, ethyl, n-propyl, isopropyl, n-butyl, iso-butyl, sec-butyl, tert-butyl and the like. The aliphatic hydrocarbon may be saturated or unsaturated. When it is unsaturated, it may contain one or more unsaturations, i.e., -C=C-double and/or -C≡C-triple bonds. If there is more than one unsaturation, the unsaturations may be conjugated or isolated. Thus, for the purpose of the specification the term "alkyl" encompasses saturated hydrocarbons as well as alkenyl, alkynyl and alkenynyl residues. "Alkenyl" preferably refers to an alkyl group, as defined above, consisting of at least two carbon atoms and at least one carbon-carbon double bond e.g., ethenyl, propenyl, butenyl or pentenyl and their structural isomeric forms such as 1- or 2-propenyl, 1-, 2-, or 3-butenyl and the like. "Alkynyl" preferably refers to an alkyl group, as defined above, consisting of at least two carbon atoms and at least one carboncarbon triple bond e. g., acetylene, ethynyl, propynyl, butynyl, or pentynyl and their structural isomeric forms as described above. Alkyl may be substituted or unsubstituted. When substituted, the substituent group(s) is one or more, for example one or two groups, individually selected from the group consisting of -C₃-C₈ cycloalkyl, -C₆-C₁₄ aryl; a 5-10 membered -heteroaryl wherein 1 to 4 ring atoms are independently selected from N, O or S: a 5-10 membered heterocycloalkyl wherein 1 to 3 ring atoms are independently selected from N, O or S; -OH; -O-C₁-C₁₀ alkyl (= C₁-C₁₀ alkoxy); -O-C₆-C₈ cycloalkyl (= C₃C₈ cycloalkoxy); -O-C₆-C₁₄ aryl (= C₆-C₁₄ aryloxy); -SH; -S-C₁-C₁₀ alkyl (= alkylthio); -S-C₆C₁₄aryl (= C₆-C₁₄ arylthio); -CN; -halo; -carbonyl; -thiocarbonyl; -O-carbamyl; -N-carbamyl; -O-thiocarbamyl; -N-thiocarbamyl; -C-amido; -N-amido; -C-carboxy; -O-carboxy; -NO₂; - silyl; -sulfinyl; -sulfonyl; and -NR⁷R⁸ where R⁷ and R⁸ are independently selected from the' group consisting of -H, -C₁-C₄ alkyl, -C₃-C₈ cycloalkyl, -C₆-C₁₄ aryl, -carbonyl, -acetyl, - sulfonyl, -amino, and trifluoromethanesulfonyl; or R⁷ and R⁶, together with the nitrogen atom to which they are attached, combine to form a five- or six-membered heterocycloalkyl ring. Preferably, the substituent(s) is/are independently selected from - chloro, -fluoro, -bromo, -hydroxy, -methoxy, -nitro, -carboxy, -methoxycarbonyl, -sulfonyl, or -amino.
Unless expressly stated otherwise, "cycloalkyl" preferably refers to cyclic hydrocarbon residue that contains no heteroatoms as ring members and that is not aromatic. "Cycloalkyl" may encompass a single cycle or more than one cycle. Preferably, cycloalkyl has 3 to 8 carbon atoms (-C₃-C₈ cycloalkyl). Cycloalkyl may be saturated, e.g., cyclopropane, cyclobutane, cyclopentane, cyclohexane, cycloheptane, adamantane; or unsaturated (e.g., cycloalkenyl, cycloalkynyl), e.g., cyclobutenyl, cyclopentenyl, cyclohexenyl, cyclohexadiene, cycloheptatriene and the like. Cycloalkyl may be substituted or unsubstituted. When substituted, the substituent group(s) is one or more, for example one or two groups, individually selected from -C₁-C₁₀ alkyl; -C₃-C₆ cycloalkyl; - C₆-C₁₄ aryl; 5-10 membered -heteroaryl wherein 1 to 4 ring atoms are independently selected from N, O or S; 5-10 membered -heterocycloalkyl wherein 1 to 3 ring atoms are independently selected from N, O or S; -OH; -O-C₁-C₁₀ alkyl; -O-C₃-C₈ cycloalkyl; -O-C₆C₁₄ aryl; -SH; -S-C₁-C₁₀ alkyl; -S-C₆-C₁₄ aryl; -CN; -halo; -carbonyl; -thiocarbonyl; -Ocarbamyl; -N-carbamyl; -O-thiocarbamyl; -N-thiocarbamyl; -C-amido; -N-amido; -Ccarboxy; -O-carboxy; -NO₂; -silyl; -sulfinyl; -sulfonyl; and -NR⁷R⁸ where R⁷ and R⁸ are independently selected from the group consisting of -H, -C₁-C₄ alkyl. -C₃-C₈ cycloalkyl, - G₆-C₁₄ aryl, -carbonyl, -acetyl, -sulfonyl, -amino, and trifluoromethanesulfonyl; or R⁷ and R⁸, together with the nitrogen atom to which they are attached, combine to form a five- or six-membered heterocycloalkyl ring. Preferably, the substituent(s) is/are independently selected from -chloro, -fluoro, -bromo, -methyl, -ethyl, -hydroxy, -methoxy, -nitro, -carboxy, -methoxycarbonyl, -sulfonyl, or -amino.
Unless expressly stated otherwise, "heterocycloalkyl" preferably refers to a monocyclic or fused ring of 5 to 10 ring atoms containing one, two, or three heteroatoms in the ring which are selected from the group consisting of N, O and -S(O)ₙ where n is 0-2, the remaining ring atoms being carbon. The rings may be saturated or unsaturated, i.e. the rings may have one or more double bonds. However, the rings are not aromatic (heterocycloalkyl ≠ heteroaryl). Examples, without limitation, of heterocycloalkyl groups are pyrrolidine, piperidine, piperazine, morpholine, imidazolidine, tetrahydropyridazine, tetrahydrofuran, thiomorpholine, tetrahydropyridine, and the like. Heterocycloalkyl may be substituted or unsubstituted. When substituted, the substituted group(s) is one or more, for example one, two, or three substituents, independently selected from the group consisting of -C₁-C₁₀ alkyl; -C₃-C₈ cycloalkyl; -C₆-C₁₄ aryl; 5-10 membered -heteroaryl wherein 1 to 4 ring atoms are independently selected from N, O or S; 5-10 membered - heterocycloalkyl wherein 1 to 3 ring atoms are independently selected from N, O or S; - OH; -O-C₁-C₁₀ alkyl; -O-C₃-C₈ cycloalkyl; -O-C₆-C₁₄ aryl; -SH; -S-C₁-C₁₀ alkyl; -S-C₆-C₁₄ aryl; -CN; -halo; -carbonyl; -thiocarbonyl; -O-carbamyl; -N-carbamyl; -O-thiocarbamyl; -N-thiocarbamyl; -C-amido; -N-amido; -C-carboxy; -O-carboxy; -NO₂; -silyl; -sulfinyl; -sulfonyl; and -NR⁷R⁸ where R⁷ and R⁸ are independently selected from the group consisting of -H, - C₁-C₄ alkyl, -C₃-C₈ cycloalkyl, -C₆-C₁₄ aryl, -carbonyl, -acetyl, -sulfonyl, -amino, and trifluoromethanesulfonyl; or R⁷ and R⁸, together with the nitrogen atom to which they are attached, combine to form a five- or six-membered heterocycloalkyl ring. Preferably, the substituent(s) is/are independently selected from -chloro, -fluoro, -bromo, -methyl, -ethyl,hydroxy, -methoxy, -nitro, -carboxy, -methoxycarbonyl, -sulfonyl, or -amino.
Unless expressly stated otherwise, "aryl" preferably refers to an aromatic all-carbon monocyclic or fused-ring polycyclic group (i.e., rings which share adjacent pairs of carbon atoms) of 6 to 14 ring atoms and having a completely conjugated pi-electron system. Examples, without limitation, of aryl groups are phenyl, naphthalenyl and anthracenyl. The aryl group may be substituted or unsubstituted. When substituted, the substituted group(s) is one or more, for example one, two, or three substituents, independently selected from the group consisting of-C₁-C₁₀ alkyl; -C₃-C₆ cycloalkyl; -C₆-C₁₄ aryl; 5-10 membered - heteroaryl wherein 1 to 4 ring atoms are independently selected from N, O or S; 5-10 membered -heterocycloalkyl wherein 1 to 3 ring atoms are independently selected from N, O or S; -OH; -O-C₁-C₁₀ alkyl; -O-C₃-C₈ cycloalkyl; -O-C₆-C₁₄ aryl; -SH; -S-C₁-C₁₀ alkyl; -SC₆-C₁₄ aryl; -CN; -halo; -carbonyl; -thiocarbonyl; -O-carbamyl; -N-carbamyl; -O-thiocarbamyl; -N-thiocarbamyl; -C-amido; -N-amido; -C-carboxy; -O-carboxy; -NO₂, -silyl; - sulfinyl; -sulfonyl; and -NR⁷R⁸ where R⁷ and R⁸ are independently selected from the group consisting of -H, -C₁-C₄ alkyl, -C₃-C₆ cycloalkyl, -C₆-C₁₄ aryl, -carbonyl, -acetyl, -sulfonyl, - amino, and trifluoromethanesulfonyl; or R⁷ and R⁸, together with the nitrogen atom to which they are attached, combine to form a five- or six-membered heterocycloalkyl ring. Preferably the substituent(s) is/are independently selected from -chloro, -fluoro, -bromo, - methyl, -ethyl, -hydroxy, -methoxy, -nitro, -carboxy, -methoxycarbonyl, -sulfonyl, or - amino.
Unless expressly stated otherwise, "hHeteroaryl" preferably refers to a monocyclic or fused aromatic ring (i.e., rings which share an adjacent pair of atoms) of 5 to 10 ring atoms in which one, two, three or four ring atoms are selected from the group consisting of N, O and S and the rest being carbon. Examples, without limitation, of heteroaryl groups are pyridyl, pyrrolyl, furyl, thienyl, imidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, 1,3,4-triazinyl, 1,2,3-triazinyl, benzofuryl, isobenzofuryl, benzothienyl, benzotriazolyl, isobenzothienyl, indolyl, isoindolyl, 3H-indolyl, benzimidazolyl, benzothiazolyl, benzoxazolyl, quinolizinyl, quinazolinyl, pthalazinyl, quinoxalinyl, cinnnolinyl, napthyridinyl, quinolyl, isoquinolyl, tetrazolyl, 5,6,7,8tetrahydroquinolyl, 5, 6, 7, 8-tetra-hydroisoquinolyl, purinyl, pteridinyl, pyridinyl, pyrimidinyl, carbazolyl, xanthenyl or benzoquinolyl. The heteroaryl group may be substituted or unsubstituted. When substituted, the substituted group(s) is one or more, for example one or two substituents, independently selected from the group consisting of - C₁-C₁₀ alkyl; -C₃-C₈ cycloalkyl; -C₆-C₁₄ aryl; 5-10 membered -heteroaryl wherein 1 to 4 ring atoms are independently selected from N, O or S; 5-10 membered -heterocycloalkyl wherein 1 to 3 ring atoms are independently selected from N, O or S; -OH; -O-C₁-C₁₀ alkyl; -O-C₃-C₆ cycloalkyl; -O-C₆-C₁₄ aryl; -SH; -S-C₁-C₁₀ alkyl; -S-C₆-C₁₄ aryl; -CN; -halo; -carbonyl; -thiocarbonyl; -O-carbamyl; -N-carbamyl; -O-thiocarbamyl; -N-thiocarbamyl; -Camido; -N-amido; -C-carboxy; -O-carboxy; -NO₂; -silyl; -sulfinyl; -suffonyl; and -NR⁷R⁸ where R⁷ and R⁸ are independently selected from the group consisting of -H, -C₁-C₄ alkyl, -C₃-C₈ cycloalkyl, -C₆-C₁₄ aryl, -carbonyl, -acetyl, -sulfonyl, -amino, and trifluoromethanesulfonyl; or R⁷ and R⁸, together with the nitrogen atom to which they are attached, combine to form a five- or six-membered heterocycloalkyl ring. Preferably the substituent(s) is/are independently selected from -chloro, -fluoro, -bromo, -methyl, -ethyl, -hydroxy, -methoxy, -nitro, -carboxy, -methoxycarbonyl, -sulfonyl, or-amino.

"Hydroxy" preferably refers to an -OH group.

"Alkoxy" preferably refers to an -O-unsubstituted alkyl and -O-substituted alkyl group, as defined herein. Examples include and are not limited to -methoxy, -ethoxy, -propoxy, -butoxy, and the like.

"Cycloalkoxy" preferably refers to an -O-cycloalkyl group, as defined herein. One example is -cyclopropyloxy.

"Aryloxy" preferably refers to both an -O-aryl and an -O-heteroaryl group, as defined herein. Examples include and are not limited to -phenoxy, -napthyloxy, -pyridyloxy, -furanyloxy, and the like.

"Mercapto" preferably refers to an -SH group.

"Alkylthio" preferably refers to both an -S-alkyl and an -S-cycloalkyl group, as defined herein. Examples include and are not limited to -methylthio, -ethylthio, and the like.

"Arylthio" preferably refers to both an -S-aryl and an -S-heteroaryl group, as defined herein. Examples include and are not limited to -phenylthio, -napthylthio, -pyridylthio, -furanylthio, and the like.

"Sulfinyl" preferably refers to a -S(O)-R" group, wherein, R" is selected from the group consisting of -H; -OH; -alkyl, -cycloalkyl, -aryl, -heteroaryl (bonded through a ring carbon) and -heterocycloalkyl (bonded through a ring carbon), as defined herein.

"Sulfonyl" preferably refers to a -S(O)₂R" group wherein, R" is selected from the group consisting of -H, -OH, -alkyl, -cycloalkyl, -aryl, -heteroaryl (bonded through a ring carbon) and -heterocycloalkyl (bonded through a ring carbon), as defined herein.

"Trihalomethyl" preferably refers to a -CX₃ group wherein X is a halo group as defined herein e. g., -trifluoromethyl, -trichloromethyl, -tribromomethyl, -dichlorofluoromethyl, and the like.

"Carbonyl" preferably refers to a -C(=O)-R" group, where R" is selected from the group consisting of -H, -alkyl, -cycloalkyl, -aryl, -heteroaryl (bonded through a ring carbon) and - heterocycloalkyl (bonded through a ring carbon), as defined herein. Representative examples include and the not limited to -acetyl, -propionyl, -benzoyl, -formyl, - cyclopropylcarbonyl, -pyridinylcarbonyl, -pyrrolidin-lylcarbonyl, and the like.

"Thiocarbonyl" preferably refers to a -C(=S)-R" group, with R" as defined herein.

"C-carboxy" and "carboxy" which are used interchangeably herein preferably refer to a - C(=O)O-R" group, with R" as defined herein, e. g. -COOH, -methoxycarbonyl, - ethoxycarbonyl, -benzyloxycarbonyl, and the like.

"O-carboxy" preferably refers to a -OC(=O)R" group, with R" as defined herein, e.g. - methylcarbonyloxy, -phenylcarbonyloxy, -benzylcarbonyloxy, and the like.

"Acetyl" preferably refers to a -C(=O)CH₃ group.

"Carboxylic acid" preferably refers to a -C-carboxy group in which R" is -H.

"Halo" or "halogen" preferably refers to -fluorine, -chlorine, -bromine or -iodine.

"Cyano" preferably refers to a -CN group.

"Nitro" preferably refers to a -NO₂ group.

"O-carbamyl" preferably refers to a -OC(=O)NR⁷R⁶ group, with R⁷ and R⁸ as defined herein.

"N-carbamyl" preferably refers to a R⁸OC(=O)NR⁷- group, with R⁷ and R⁸ as defined herein.

"O-thiocarbamyl" preferably refers to a -OC(=S)NR⁷R⁸ group, with R⁷ and R⁸ as defined herein.

"N-thiocarbamyl" preferably refers to a R⁸OC(=S)NR⁷- group, with R⁷ and R⁸ as defined herein.

"Amino" preferably refers to an -NR⁷R⁸ group, wherein R⁷ and R⁸ are independently -H or unsubstituted C₁-C₆ alkyl, e.g, -NH₂, -dimethylamino, -diethylamino, -ethylamino, - methylamino, and the like.

"C-amido" preferably refers to a -C(=O)NR⁷R⁸ group, with R⁷ and R⁸ as defined herein. For example, R⁷ is -H or unsubstituted -C₁-C₄ alkyl and R⁸ is -H, -C₁-C₄ alkyl optionally substituted with -heterocycloalkyl, -hydroxy, or -amino. For example, -C(=O)NR⁷R⁸ may be -aminocarbonyl, -dimethylaminocarbonyl, -diethylaminocarbonyl, -diethylaminoethylaminocarbonyl, -ethylaminoethylaminocarbonyl, and the like.

"N-amido" preferably refers to a R⁸C(=O)NR⁷- group, with R⁷ and R⁸ as defined herein, e.g. -acetylamino, and the like.

If not expressly stated otherwise, any residue, group or moiety defined herein that can be substituted is preferably substituted with one or more substituents independently selected from the group consisting of -C₁-C₁₀ alkyl; -C₃-C₈ cycloalkyl; -C₆-C₁₄ aryl; 5-10 membered - heteroaryl wherein 1 to 4 ring atoms are independently selected from N, O or S; 5-10 membered -heterocycloalkyl wherein 1 to 3 ring atoms are independently selected from N, O or S; -OH; -O-C₁-C₁₀ alkyl; -O-C₃-C₈ cycloalkyl; -O-C₆-C₁₄ aryl; -SH; -S-C₁-C₁₀ alkyl; -SC₆-C₁₄ aryl; -CN; -halo; -carbonyl; -thiocarbonyl; -O-carbamyl; -N-carbamyl; -O-thiocarbamyl; -N-thiocarbamyl; -C-amido; -N-amido; -C-carboxy; -O-carboxy; -NO₂; -silyl; -sulfinyl; -sulfonyl; and -NR⁷R⁸ where R⁷ and R⁸ are independently selected from the group consisting of -H, -C₁-C₄ alkyl, -C₃-C₈ cycloalkyl, -C₆-C₁₄ aryl, -carbonyl, -acetyl, -sulfonyl, -amino, and trifluoromethanesulfonyl; or R⁷ and R⁸, together with the nitrogen atom to which they are attached, combine to form a five- or six-membered heterocycloalkyl ring. Preferably the substituent(s) is/are independently selected from -chloro, -fluoro, -bromo, -methyl, -ethyl, -hydroxy, -methoxy, -nitro, -carboxy, -methoxycarbonyl, -sulfonyl, or -amino.

The invention also relates to the stereoisomers of the compounds according to general formula (I), e.g. the enantiomers or diastereomers in racemic, enriched or substantially pure form.

In another aspect, the present invention relates to a pharmaceutical composition comprising any of the compounds or salts of the present invention and, optionally, a pharmaceutically acceptable carrier or excipient. This composition may additionally comprise further compounds or medicaments, such as, for example, neuroprotective or antinociceptive agents besides the compounds according to general formula (I).

"Pharmaceutical composition" preferably refers to a mixture of one or more of the compounds described herein, or physiologically/pharmaceutically acceptable salts or prodrugs thereof, with other chemical components, such as physiologically/ pharmaceutically acceptable carriers and excipients. The purpose of a pharmaceutical composition is to facilitate administration of a compound to an organism.

As used herein, a "physiologically/pharmaceutically acceptable carrier" refers to a carrier or diluent that does not cause significant irritation to an organism and does not abrogate the biological activity and properties of the administered compound.

A "pharmaceutically acceptable excipient" refers to an inert substance added to a pharmaceutical composition to further facilitate administration of a compound. Examples, without limitation, of excipients include calcium carbonate, calcium phosphate, various sugars and types of starch, cellulose derivatives, gelatin, vegetable oils and polyethylene glycols.

Physiologically or pharmaceutically acceptable carriers and excipients are known to the skilled person. In this regard it can be referred to, e.g., H.P. Fiedler, Lexikon der Hilfsstoffe für Pharmazie, Kosmetik und angrenzende technische Gebiete, Editio Cantor Aulendorf, 2001.

The pharmaceutical composition according to the invention can be, e.g., solid, liquid or pasty.

A further aspect of the invention relates to a pharmaceutical dosage form comprising the pharmaceutical composition according to the invention.

The pharmaceutical dosage form according to the invention may be adapted for various routes of administration (e.g. systemic, parenteral, topic, local), such as oral administration, infusion, injection and the like.

Pharmaceutical dosage forms that are adapted for oral administration include tablets, pellets, capsules, powders, granules and the like.

The pharmaceutical dosage form is preferably adapted for administration once daily, twice daily or thrice daily. The pharmaceutical dosage form may release the compound according to general formula (I) immediately (immediate release formulation) or over an extended period of time (retarded release, delayed release, prolonged release, sustained release, and the like).

The compounds according to the invention are mediators of the adenine receptor. They exhibit antinociceptive and neuroprotective properties.

In another aspect, the invention relates to the use of the compounds according to general formula (I) for activating or antagonizing adenine receptor function. Thus, in one embodiment, the compounds of the invention may be used as neuroprotective drugs. In a further aspect, the compounds according to general formula (I) may thus also be used for the prevention, alleviation and/or treatment of a disease or disorder related to adenine receptor activity.

The terms "diseases and disorders related to adenine receptor function", "diseases and disorders connected to adenine receptor function" and "disease or disorder related to adenine receptor activity" are used interchangeably herein to refer to a condition involving adenine receptor activity. Examples for such diseases and disorders are neurodegenerative diseases, nociceptive pain, and neuropathic pain. Since the adenine receptor is also expressed in brain, e.g. in the cortex, also included are CNS disorders, such as neuroinflammatory conditions and neurodegenerative disorders (e.g. Alzheimer's and Parkinson's disease).

"Treat", "treating" and "treatment" preferably refer to a method of alleviating or abrogating an adenine receptor related disease or disorder and/or its attendant symptoms.

"Prevent", "preventing" and "prevention" preferably refer to a method of hindering an adenine receptor related disease or disorder from occuring, i.e. a prophylactic method.

"Organism" preferably refers to any living entity comprised of at least one cell. A living organism can be as simple as, for example, a single eukariotic cell or as complex as a mammal, including a human being.

"Therapeutically effective amount" preferably refers to that amount of the compound being administered which will relieve to some extent one or more of the symptoms of the disorder being treated.

Preferably, the subject afflicted by a disease treated, alleviated or prevented according to the invented use is a human.

A further aspect of the invention relates to the use of a compound according to general formula (I) for the manufacture of a pharmaceutical composition according to the invention or of a pharmaceutical dosage form according to the invention for preventing, ameliorating or treating pain or a neurodegenerative disease.

Preferably, the neurodegenerative disease is selected from the group consisting of Alzheimer's disease; multiple sclerosis; Huntington's disease; Parkinson's disease; AIDS related dementia; amyotrophic lateral sclerosis; a retinal disease; epilepsy; stroke; acute thromboembolic stroke, focal ischemia, global ischemia, or transient ischemic attack; ischemia resulting from a surgical technique involving prolonged halt of blood flow to the brain; head trauma; spinal trauma; optic nerve stroke; anterior ischemic optic neuropathy; traumatic optic neuropathy; glaucoma; optic neuritis; compressive optic neuropathy; hereditary neuropathy; and schizophrenia.

In still another aspect, the invention relates to a method for activating or antagonizing the adenine receptor function comprising the step of contacting cells expressing an adenine receptor with at least one of the above compounds. This method can include a method for the treatment of a disease or disorder mediated by or involving an adenine receptor, the method comprising the administration of a pharmaceutically active amount of at least one of the compounds according to the invention to a patient in need thereof.

The compounds according to general formula (I) may be synthesized by conventional synthetic methods starting from building blocks that are commercially available.

In a further aspect, the invention relates to a method of preparing a compound of general formula (I).

In a preferred embodiment, the method comprises the preparation of N⁶-(omegaaminoacyl)adenine derivatives of general Formula (II) 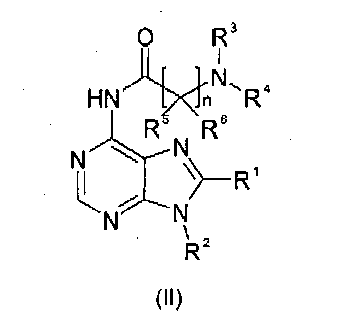 by reacting adenine derivatives of general formula (III) with amino acids of general formula (IV) 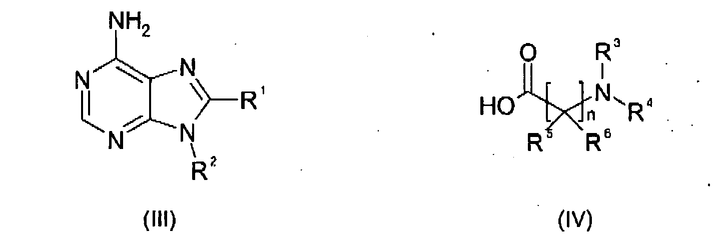 wherein n, R¹, R², R³, R⁴, R⁵, and R⁶ are defined as above.

In one embodiment of the reaction, dicyclohexyl carbodiimide (DCC) is used as amide coupling reagent.

In a further preferred embodiment of the reaction, the N-protected amino acid having general formula (IV) is activated as a mixed anhydride.

In another preferred embodiment of the reaction, (benzotriazol-1-yloxy)tripyrrolidinophosphonium hexafluorophosphate (PyBOP) and N-hydroxybenzotriazole (HOBt) are used as amide coupling reagents.

In still another preferred embodiment of the reaction, 2-(6-chloro-1 H-benzotriazol-1-yl)1,1,3,3-tetramethylaminium hexafluorophosphate (HCTU) and N-hydroxybenzotriazole (HOBt) are used as amide coupling reagents.

In another aspect, the present invention relates to a synthetic method for the deprotection of compounds of general formula (II), for example the cleavage of R³, being a *tert*butyloxycarbonyl (*t*-Boc) protecting group, by strong acid catalysis, affording compounds of general formula (V)

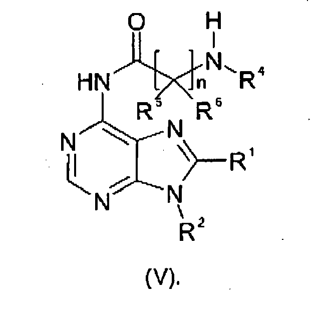

The only known strategy for the selective acylation of the exocyclic amino function of adenine with amino acids utilizes an amide coupling of carbobenzoxyamino acids to the exocyclic amino function of adenine with-N,N'-dicyclohexylcarbodiimide (DCC) in dimethylsulfoxide as the central step (Scheme 1) 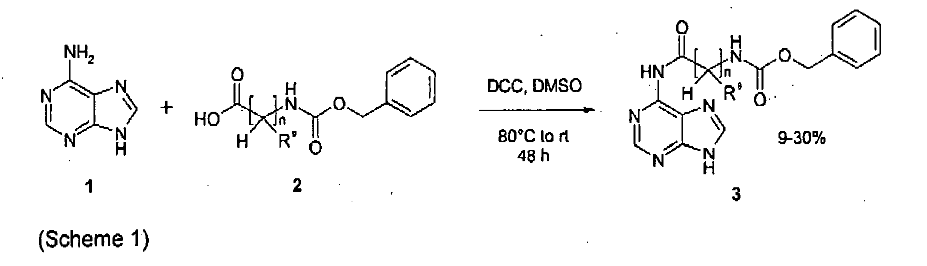 wherein R⁹ is -H, -CH₃, -CH(CH₃)₂, -CH₂CH(CH₃)₂, -CH(CH₃)C₂H₅, and -benzyl (Brink, J. J. et al., J. Med. Chem., 1963, 6, 563).

However, this study was limited to α-aminoacyl derivatives with a chain length of n = 1, which decompose at room temperature under neutral aqueous conditions after an elucidated mechanism of rearrangement (Chheda, G. B. et al., J. Org. Chem., 1969, 34, 3498).

Moreover, major drawbacks of this reaction are poor yields and the requirement of harsh reaction conditions, e.g. high temperatures and long reaction times.

It has been surprisingly found that the activation of the amino acid as a mixed anhydride (method B) provided slightly higher yields and the use of PyBOP /HOBt (method C) or HCTU / HOBt (method D) as coupling reagents provided considerably higher yields of the desired amides when compared to the method using DCC (method A).

Moreover, the methods B. C, and D according to the invention are characterized by mild reaction conditions and shorter reaction times, providing amide formation at room temperature within 5 - 12 hours (Scheme 2). 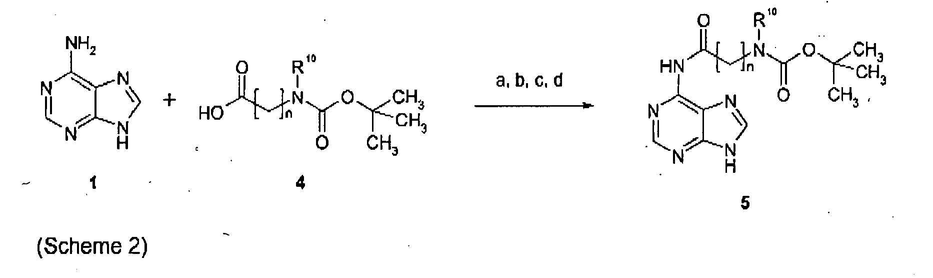 wherein n is 2 and 3, and R¹⁰ is -methyl, -ethyl, -propyl, -hexyl, -benzyl, and -phenethyl.
(a) reaction in the presence of DCC in dimethylsulfoxide (DMSO) at 80 °C and cooling to room temperature for 48 h (method A, yields 10 - 22%);
(b) reaction in the presence of isobutylchloroformiate and N-methylmorpholine (NMM) in dimethylformamide (DMF) at -25 °C to room temperature for 5 h (method B, yields 13 - 29%);
(c) reaction in the presence of PyBOP, HOBt, and diisopropylethylamine (DIPEA) in DMF at room temperature for 12 h (method C, yields 29 - 40%);
(d) reaction in the presence of HCTU, HOBt and DIPEA in DMF at room temperature for 12 h (method D, yields 27 - 32%);

In another aspect, the invention relates to a method to deprotect N-boc protected N⁶(omega-aminoacyl)adenine derivatives having the general formula of 5. Acidic cleavage of the N-boc protection group, for example with HCl (4.0 M in dioxane) yields hydrochlorides of the primary or secondary amines of the general formula of 6 that exhibit excellent water solubility, which is a basic need for the design of bioavailable drugs. 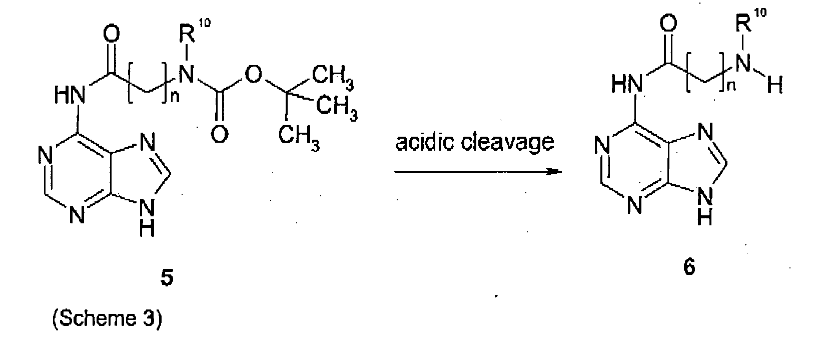 wherein n and R¹⁰ are defined as above.

Typical reaction conditions for this deprotection include, e.g.:
(a) HCl (in dioxane), room temperature, 15 min
(b) TFA, dichloromethane, room temperature, 1 h

The present invention thus relates to an improved method for the synthesis of the compounds of general formula (I), which includes a condensation step according to method B, C, and D or the deprotection of N-protected derivatives 5.

The inventions illustratively described herein may suitably be practiced in the absence of any element or elements, limitation or limitations, not specifically disclosed herein. Thus, for example, the term "includes" shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied therein herein disclosed may be resorted to by those skilled in the art, and that such modifications and variations are considered to be within the scope of this invention.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

Other embodiments are within the following claims and non-limiting examples. In addition, where features or aspects of the invention are described in terms of Markush groups, those skilled in the art will recognize that the invention is also thereby described in terms of any individual member or subgroup of members of the Markush group.

The present inventions will be explained in more detail in the following examples. However, the examples are only used for illustration and do not limit the scope of the present invention.

All commercially available reagents were obtained from various producers (Acros, Aldrich, Fluka, Merck, Sigma, and Novabiochem) and used without further purification. Solvents were used without additional purification or drying, unless otherwise noted. Thin-layer chromatography was performed using TLC aluminum sheets silica gel 60 F₂₅₄, or TLC aluminum sheets RP silica gel 18 F₂₅₄ (Merck, Darmstadt, Germany). Column chromatography was carried out with silica gel 0.060-0.200 mm, pore diameter ca 6 nm. ESI-mass spectra were obtained on an LCMS instrument (Applied Biosystems API 2000 LCMS/MS, HPLC Agilent 1100) using the following procedure: the compounds were dissolved at a concentration of 0.5 mg/ml in H₂O : MeOH = 9 : 1, containing 2 mM NH₄CH₃COO. Then, 10 µl of the sample was injected into an HPLC column (Phenomenix Luna 3µ C18, 50 x 2.00 mm). Elution was performed with a gradient of water : methanol (containing 2 mM NH₄CH₃COO) from 90 : 10 to 0 : 100 for 30 min at a flow rate of 250 µl/min, starting the gradient after 10 min. UV absorption was detected from 200 to 950 nm using a diode array detector. Purity of the compounds was determined at 254 nm. Elemental microanalyses were performed on a VarioEL apparatus. ¹H-, and ¹³C-NMR spectra were obtained on a Bruker Avance 500 MHz NMR spectrometer at 500 MHz (¹H), or 126 MHz (¹³C), respectively. CDCl₃, DMSO-d₆, CD₃OD, or D₂O were used as solvents as indicated below. Chemical shifts are reported in parts per million (ppm) relative to the deuterated solvent. Coupling constants J are given in Hertz and spin multiplicities are given as s (singlet), d (doublet), t (triplet), q (quartet), m (multiplet), br (broad). NMR spectra were recorded at room temperature, unless otherwise noted. Melting points were determined on a Büchi B-545 melting point apparatus and are uncorrected. A freeze dryer (CHRIST ALPHA 1-4 LSC) was used for lyophilization. Reactions were monitored by TLC on silica gel 60 F₂₅₄ (Merck) aluminium plates.

### Example 1: General synthesis procedure

### General procedure A: Synthesis of N-alkylated amino acids

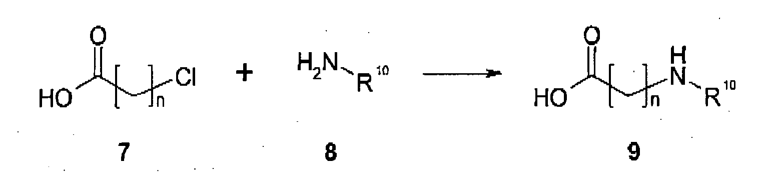

wherein n and R¹⁰ are defined as above.

The appropriate omega-chlorocarboxylic acid (53 mmol) was added portionwise to the ice cooled amine (1 mol). Methylamine (40%) and ethylamine (70%) were applied as aqueous solutions. The solution was stirred at room temperature overnight, NaOH (4.7 g, 0.12 mol) was added and the mixture was evaporated to dryness and coevaporated with water (3x 50 ml). In cases of hexylamine, benzylamine, and phenethylamine the residue was dissolved in water (15 ml) and remaining amine was removed by extraction with diethyl ether (3 x 50 ml). The aqueous layer was evaporated to dryness. The resulting N-alkylated amino acid was directly used for the following N-boc protection.

### General procedure B: N-Boc protection of N-alkylated omega-amino acids

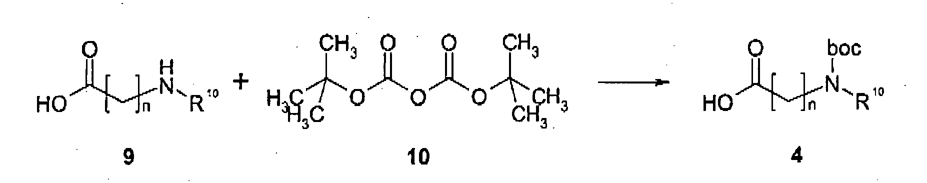

wherein n and R¹⁰ are defined as above.

The N-alkylated amino acid was dissolved in a mixture of water (75 ml) and dioxane (75 ml), di-*tert*-butyl dicarbonate (53 mmol) was added portionwise, and the solution was stirred at room temperature for 2 h, heated at reflux for 30 min and cooled to room temperature. The pH was adjusted to 1-2 by addition of NaHSO₄ (10% in water) and the product was extracted with ethyl acetate (4 x 100 ml). The organic layer was dried (Na₂SO₄) and ethyl acetate was evaporated under reduced pressure. Colorless crystals were obtained by storage of the resulting oil overnight at -20 °C.

### General procedure C: Amide coupling reactions of N-boc protected N-alkylated omegaamino acids to adenine

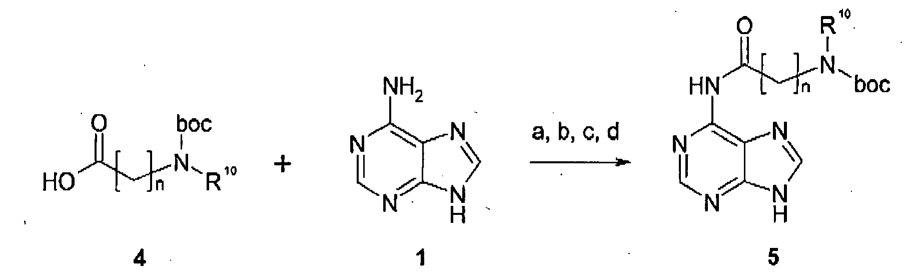

wherein n and R¹⁰ are defined as above.
(a) Adenine (0.27 g, 2.0 mmol), carboxylic acid (2.0 mmol), and DCC (2.0 mmol) were dissolved in 10 ml DMSO at 80 °C and stirred at room temperature for 48 h. The precipitated dicyclohexyl urea was filtered off and the filtrate was evaporated to dryness under reduced pressure. The product was separated from unreacted adenine by RPHPLC (method A).
(b) Carboxylic acid (2.0 mmol) and N-methylmorpholine (2.0 mmol) were dissolved in 10 ml dry DMF and were cooled at -25 °C. Isobutylchloroformiate (0.26 ml, 2.0 mmol) was added and 40 sec later adenine (0.54 g, 4.0 mmol) was added. All steps were performed under argon. The suspension was stirred under argon at -25 °C for 2 h and at room temperature for 3 h. DMF was evaporated under reduced pressure. The product was purified by column chromatography on silica gel eluting with 5% methanol in DCM. The appropriate fractions were separated from remaining adenine by RP-HPLC (method B).
(c) Carboxylic acid (2.0 mmol), PyBOP (1.14 g, 2.2 mmol), and HOBt (0.30 g, 2.2 mmol) were dissolved in dry DMF (6 ml). DIPEA (0.30 ml, 2.2 mmol) was added and 1 min later adenine (0.54 g, 4.0 mmol) was added. All steps were performed under argon. The suspension was stirred under argon at room temperature overnight. DMF was evaporated under reduced pressure. The product was purified by column chromatography on silica gel eluting with 5% methanol in DCM. The appropriate fractions were separated from remaining adenine by RP-HPLC (method C).
(d) This method corresponds to method C, but HCTU (0.91 g, 2.2 mmol) was used instead of PyBOP (method D).

### General procedure D: Deprotection of N-boc protected N-alkylated N⁶-(omegaaminoacyl)adenine derivatives

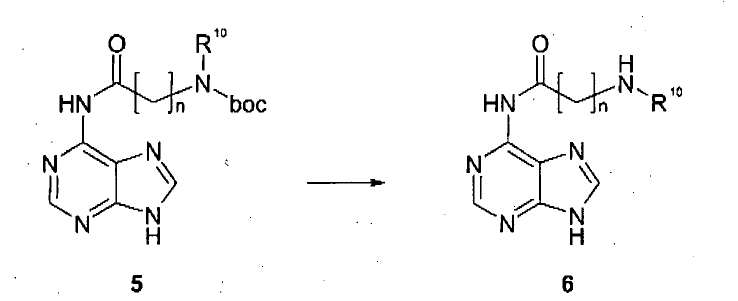

wherein n and R¹⁰ are defined as above.

The N-boc protected compound (0.1 mmol) was suspended in 2 ml HCl (4.0 M in dioxane) and the mixture was stirred at room temperature for 15 min. Diethyl ether (10 ml) was added and the resulting crystals were filtered and washed with diethyl ether. The crystals were dissolved in water (10 ml), filtered, and lyophilized.

### Example 2: 3-(tert-Butyloxycarbonyl-methyl-amino)-N(9H-purin-6-yl)propionamide.

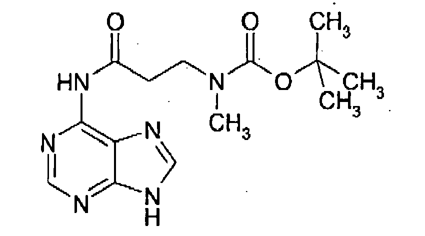

*Reaction conditions:* According to general procedure C (2.0 mmol scale) in 10.2% yield (method A), 12.5% yield (method B), 32.7% yield (method C), and 26.9% yield (method D). RP-HPLC was performed using a gradient from 25% methanol in water to 100% methanol.
*Analytical data:* mp 205 °C. ¹H-NMR (500 MHz, DMSO-d₆, 2 isomers): δ 1.29 (s, 9H), 2.75 (t, 2H, *J* = 6.85 Hz), 2.82 (s, 3H), 3.53 (t, 2H, *J* = 6.85 Hz), 8.39 (s, 1 H), 8.62 (s, 1H), 11.21 (s (br), 1 H), 12.18 (s (br), 1H). ¹³C-NMR (125 MHz, DMSO-d₆, 2 isomers) 6 28.07, 34.27, 34.84, 44.94, 78.71, 113.86, 144.33, 145.68, 151.35, 154.70, 161.34, 171.69. LCMS (m/z): 319 ([M-H]⁻), 321 ([M+H]⁺). Anal. (C₁₄H₂₀N₆O₈ · 0.55 H₂O) C, H, N. Purity by HPLC-UV (254 nm)-ESI-MS: 99%.

### Example 3: 3-(tert-Bufyloxycarbonyl-ethyl-amino)-N(9H-purin-6-yl)propionamide.

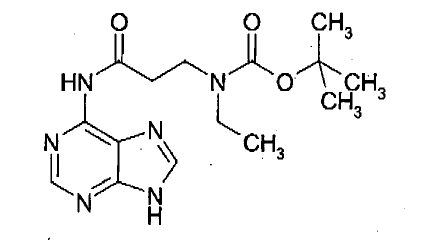

*Reaction conditions:* According to general procedure C (2.0 mmol scale) in 31.1 % yield (method C). RP-HPLC was performed using a gradient from 25% methanol in water to 100% methanol.
*Analytical* data: mp 198 °C. ¹H-NMR (500 MHz, DMSO-d₆, 2 isomers) δ 1.04 (t, 3H, *J* = 6.95 Hz), 1.34 (s, 9H), 2.76 (t, 2H, *J* = 7.08 Hz), 3.21 (quart, 2H, *J* = 6.95 Hz), 3.49 (t, 2H, *J* = 7.08 Hz), 8.39 (s, 1 H), 8.62 (s, 1H), 11.18 (s (br), 1H), 12.19 (s (br), 1H). ¹³C-NMR (125 MHz, DMSO-d₆, 2 isomers) δ 13.67, 28.14, 35.61, 41.68, 42.79, 78.66, 113.93, 144.38, 145.69, 151.36, 154.56, 161.66, 171.68. LC-MS (m/z): 333 ([M-H]⁻), 335 ([M+H]⁺). Anal. (C₁₅H₂₂NaO₃ · 0.4 H₂O) C, H, N. Purity by HPLC-UV (254 nm)-ESI-MS: 100%.

### Example 4: 3-(tert-Butyloxycarbonyl-propyl-amino)-N(9H-purin-6-yl)propionamide.

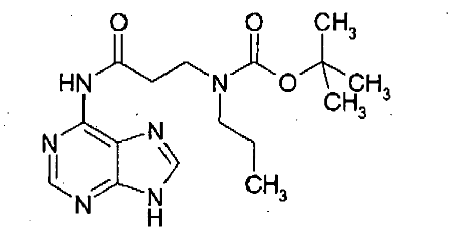

*Reaction conditions:* According to general procedure C (2.0 mmol scale) in 34.2% yield (method C). RP-HPLC was performed using a gradient from 35% methanol in water to 100% methanol.
*Analytical data:* mp 205 °C. ¹H-NMR (500 MHz, DMSO-d₆, 2 isomers) δ 0.81 (t, 3H, *J* = 7.40 Hz), 1.33 (s, 9H), 1.48 (m, 2H), 2.76 (t, 2H, *J* = 7.10 Hz), 3.14 (t, 2H, *J* = 7.25. Hz), 3.49 (t, 2H, *J* = 7.10 Hz), 8.39 (s, 1H), 8.61 (s, 1H), 11.17 (s (br), 1H), 12.14 (s (br), 1H). ¹³C-NMR (125 MHz, DMSO-d₆, 2 isomers) δ 11.25, 21.36, 28.13, 35.47, 43.16, 48.49, 78.67, 114.00, 144.39, 145.70, 151.38, 154.73, 161.40, 171.70. LC-MS (m/z): 347 ([M-H]⁻ ), 349 ([M+H]⁺). Anal. (C₁₆H₂₄N₆O₃ · 0.25 H₂O) C, H, N. Purity by HPLC-UV (254 nm)-ESIMS: 99%.

### Example 5: 3-(tert-Butyloxycarbonyl-hexyl-amino)-N(9H-purin-6-yl)propionamide.

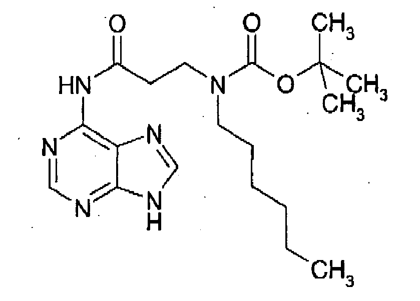

*Reaction conditions:* According to general procedure C (2.0 mmol scale) in 29.3% yield (method C). RP-HPLC was performed using a gradient from 60% methanol in water to 100% methanol.
*Analytical data:* mp 185 °C. ¹H-NMR (500 MHz, DMSO-d₆, 2 isomers) δ 0.82 (t, 3H, *J* = 6.95 Hz), 1.22 (m, 6H), 1.34 (s, 9H), 1.45 (m, 2H), 2.76 (t, 2H, *J* = 6.95 Hz), 3.16 (t, 2H, *J* = 7.40 Hz), 3.49 (t, 2H, *J* = 6.95 Hz), 8.39 (s, 1H), 8.61 (s, 1 H), 11.17 (s (br), 1 H), 12.19 (s (br), 1H). ¹³C-NMR (125 MHz, DMSO-d₆, 2 isomers) δ 13.95, 22.15, 25.99, 27.83, 28.11, 31.06, 35.49, 43.11, 46.81, 78.63, 113.89, 144.27, 145.70, 151.32, 154.67, 161.54, 171.68. LC-MS (m/z): 389 ([M-H]⁻), 391 ([M⁺H]⁺). Anal. (C₁₉H₃₀N₆O₃ · 0.6 H₂O) C, H, N. Purity by HPLC-UV (254 nm)-ESI-MS: 97%.

### Example 6: 3-(tert-Butyloxycarbonyl-benzyl-amino)-N(9H-purin-6-yl)propionamide.

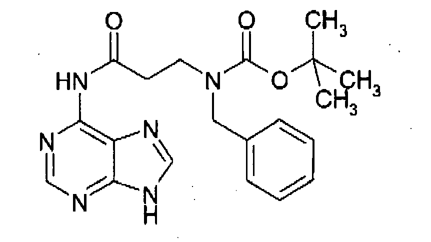

*Reaction conditions:* According to general procedure C (2.0 mmol scale) in 34.4% yield (method C). RP-HPLC was performed using a gradient from 50% methanol in water to 100% methanol.
*Analytical data:* mp 202 °C. ¹H-NMR (500 MHz, DMSO-d₆, 2 isomers) δ 1.36 (s, 9H), 2.79 (t, 2H, *J* = 6.93 Hz), 3.47 (s (br), 2H), 4.44 (s, 2H), 7.22 - 7.24 (m, 3H), 7.31 - 7.34 (m, 2H), 8.39 (s, 1H), 8.61 (s, 1H), 11.24 (s (br), 1H), 12.06 (s (br), 1H). ¹³C-NMR (125 MHz, DMSO-d₆, 2 isomers) δ 28.06, 35.07, 42.90, 49.93, 79.22, 113.14, 127.13, 127.35, 128.56, 138.52, 143.94, 145.88, 151.34, 155.01, 162.16, 171.69. LC-MS (m/z): 395 ([MH]⁻), 397 ([M+H]⁺). Anal. (C₂₀H₂₄N₆O₃ · 0.45 H₂O) C, H, N. Purity by HPLC-UV (254 nm)ESI-MS: 100%.

### Example 7: 3-(tert-Butyloxycarbonyl-phenethyl-amino)-N(9H-purin-6-yl)propionamide.

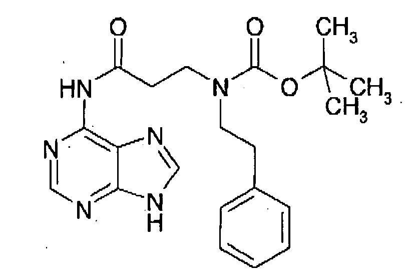

*Reaction conditions:* According to general procedure C (2.0 mmol scale) in 35.2% yield (method C). RP-HPLC was performed using a gradient from 60% methanol in water to 100% methanol.
*Analytical data:* mp 162 °C. ¹H-NMR (500 MHz, DMSO-d₆, 2 isomers) δ 1.30 (s, 9H), 2.77 (m, 4H), 3.40 (t, 2H, J = 7.55 Hz), 3.47 (s, 2H), 7.18 - 7.19 (m, 3H), 7.26 - 7.29 (m, 2H), 8.39 (s, 1H), 8.61 (s, 1 H), 11.17 (s (br), 1H), 12.15 (s (br), 1H). ¹³C-NMR (125 MHz, DMSO-d₆, 2 isomers) δ 28.05, 34.35, 35.49, 43.38, 48.80, 78.78, 113.38, 126.25, 128.46, 128.90, 139.31, 144.31, 144.61, 145.71, 151.37, 154.54, 171.69. LC-MS (m/z): 409 ([MH]⁻), 411 ([M+H]⁺). Anal. (C₂₁H₂₆N₆O₃ · 0.5 H₂O) C, H, N. Purity by HPLC-UV (254 nm)ESI-MS: 100%.

### Example 8: 3-tert-Butyloxycarbonylamino-N(9H-purin-6-yl)propionamide.

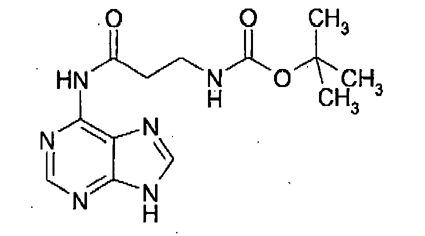

*Reaction conditions:* According to general procedure C (8.0 mmol scale) in 28.5% yield (method B). RP-HPLC was performed using a gradient from 25% methanol in water to 100% methanol.
*Analytical data:* mp 191 °C. ¹H-NMR (500 MHz, DMSO-d₆) δ 1.35 (s, 9H), 2.68 (t, 2H, *J* = 6.93 Hz), 3.29 (m, 2H), 6.85 (s (br), 1 H), 8.41 (s, 1 H), 8.61 (s, 1H), 11.11 (s (br), 1H), 12.15 (s (br), 1H). ¹³C-NMR (125 MHz, DMSO-d₆) δ 28.35, 36.31, 77.84, 113.80, 144.25, 145.65, 151.35, 155.66, 161.92, 171.62 (1 CH₂ signal not detectable). LC-MS (m/z): 305 ([M-H]⁻), 307 ([M+H]⁺). Anal Anal. (C₁₃H₁₈N₆O₃ · 0.8 H₂O) C, H, N. Purity by HPLC-UV (254 nm)-ESI-MS: 99%.

### Example 9: 4-tert-Butyloxycarbonylamino-N(9H-purin-6-yl)butyramide.

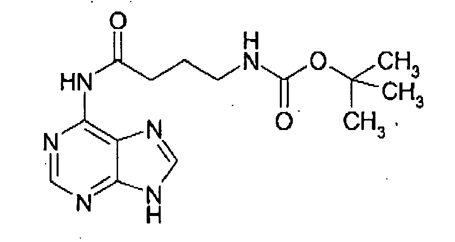

*Reaction conditions:* According to general procedure C (4.0 mmol scale) in 16.7% yield (method A), 31.9% yield (method C). RP-HPLC was performed using a gradient from 30% methanol in water to 100% methanol.
*Analytical data:* mp 223 °C. ¹H-NMR (500 MHz, DMSO-d₆) δ 1.34 (s, 9H), 1.75 (m, 2H), 2.53 (t, 2H, *J* = 7.25), 2.99 (quart, 2H, *J* = 6.52 Hz), 6.81 (s (br), 1 H), 8.38 (s, 1 H), 8.60 (s, 1H), 11.04 (s (br), 1H), 12.21 (s (br), 1 H). ¹³C-NMR (125 MHz, DMSO-d₆) δ 25.23, 28.37, 33.23, 39.50, 77.60, 114.26, 144.52, 145.74, 151.28, 155.74, 161.47, 173.02. LC-MS (m/z): 319 ([M-H]⁻), 321 ([M+H]⁺). Anal. (C₁₄H₂₀N₆O₃ · 0.8 H₂O) C, H, N. Purity by HPLCUV (254 nm)-ESI-MS: 99%.

### Example 10: 3-Methyl-amino-N(9H-purin-6-yl)propionamide dihydrochloride.

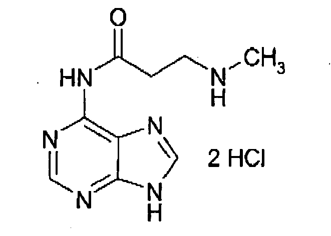

*Reaction conditions:* According to general procedure D (0.16 mmol scale) in 94.6% yield. *Analytical data:* mp 213 °C. ¹H-NMR (500 MHz, DMSO-d₆) δ 2.58 (t, 3H, *J* = 5.50 Hz), 3.06 (t, 2H, *J* = 6.78 Hz), 3.22 (m, 2H), 8.74 (s, 1H), 8.78 (s, 1H), 9.08 (s (br), 2H), 11.60 (s (br), 1H). ¹³C-NMR (125 MHz, DMSO-d₆) δ 32.06, 32.70, 43.80, 114.65, 144.90, 145.22, 151.55, 158.38, 170.33. LC-MS (m/z): 219 ([M-H]⁻), 221 ((M+H]⁺). Anal. (C₉H₁₂N₆O · 2 HCl · 1 H₂O) C, H, N. Purity by HPLC-UV (254 nm)-ESI-MS: 100%.

### Example 11: 3-Ethyl-amino-N(9H-purin-6-yl)propionamide dihydrochloride.

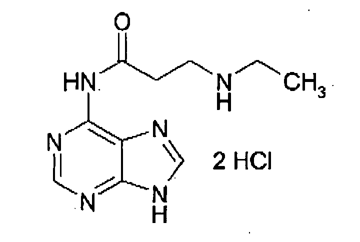

*Reaction conditions:* According to general procedure D (0.16 mmol scale) in 96.2% yield. *Analytical data:* mp 216 °C. ¹H-NMR (500 MHz, DMSO-d₆) δ 1.24 (t, 3H, J = 7.25 Hz), 2.97 (m, 2H), 3.07 (t, 2H, J = 6.78 Hz), 3.22 (m, 2H), 8.73 (s, 1H), 8.75 (s, 1H), 9.13 (s (br), 2H), 11.59 (s (br), 1H). ¹³C-NMR (125 MHz, DMSO-d₆) δ 11.03, 32.19, 41.72, 42.20, 114.64, 144.85, 145.24, 151.51, 158.58, 170.23. LC-MS (m/z): 233 ([M-H]⁻), 235 ([M+H]⁺). Anal. (C₁₀H₁₄N₆O · 2 HCl · 0.4 H₂O) C, H, N. Purity by HPLC-UV (254 nm)-ESI-MS: 100%.

### Example 12: 3-Propyl-amino-N(9H-purin-6-y/)propionamide dihydrochloride.

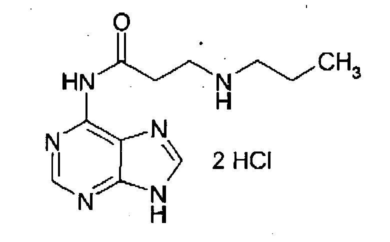

*Reaction conditions:* According to general procedure D (0.16 mmol scale) in 94.9% yield. *Analytical data:* mp 171 °C. ¹H-NMR (500 MHz, DMSO-d₆) δ 0.93 (s, 9H), 1.67 (m, 2H), 2.89 (m, 2H), 3.07 (t, 2H, J = 6.90 Hz), 3.23 (m, 2H), 8.69 (s, 1H), 8.71 (s, 1H), 9.06 (s (br), 2H), 11.53 (s (br), 1H). ¹³C-NMR (125 MHz, DMSO-d₆) δ 11.12, 19.03, 32.14, 42.22, 48.65, 114.70, 144.77, 145.31, 151.47, 159.06. 170.18. LC-MS (m/z): 247 ([M-H]⁻), 249 ([M⁺H]+). Anal. (C₁₁H₁₆N₆O · 2 HCl · 1.25 H₂O) C, H, N. Purity by HPLC-UV (254 nm)-ESIMS: 96

### Example 13: 3-Benzyl-amino-N(9H-purin-6-yl)propionamide dihydrochloride.

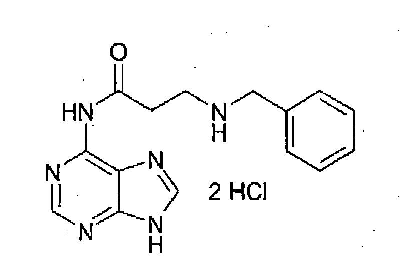

*Reaction conditions:* According to general procedure D (0.16 mmol scale) in 97.6% yield. *Analytical data:* mp 197 °C. ¹H-NMR (500 MHz, DMSO-d₆) δ 3.10 (t, 2H, *J* = 7.08 Hz), 3.22 (m, 2H), 4.19 (t, 2H, *J* = 5.35 Hz), 7.39 - 7.46 (m, 3H), 7.58 - 7.61 (m, 2H), 8.63 (s, 1 H), 8.69 (s, 1 H), 9.52 (s (br), 2H), 11.48 (s (br), 1 H). ¹³C-NMR (125 MHz, DMSO-d₆) δ 32.12, 41.85, 50.19, 114.72, 128.81, 129.08, 130.24, 131.97, 144.67, 145-39, 151.44, 159.46, 170.08. LC-MS (m/z): 295 ([M-H]⁻), 297 ([M+H]⁺), Anal. (C₁₅H₁₆N₆O · 2 HCl) C, H, N. Purity by HPLC-UV (254 nm)-ESI-MS: 99%.

### Example 14: 3-Phenethyl-amino-N(9H-purin-6-yl)propionamide dihydrochloride.

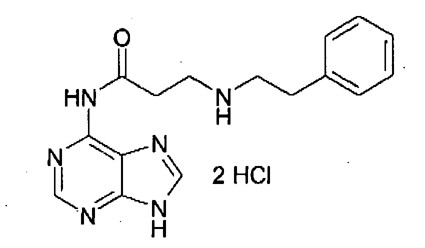

*Reaction conditions:* According to general procedure D (0.16 mmol scale) in 98.6% yield. *Analytical data:* mp 196 °C. ¹H-NMR (500 MHz, DMSO-d₆) δ 3.03 (m, 2H), 3.10 (t, 2H, J = 6.93 Hz), 3.19 (m, 2H), 3.29 (m, 2H), 7.23 - 7.29 (m, 3H), 7.32 - 7.35 (m, 2H), 8.72 (s, 1H), 8.73 (s, 1 H), 9.34 (s (br), 2H), 11.57 (s (br), 1H). ¹³C-NMR, (125 MHz, DMSO-d₆) δ 31.59, 32.16, 42.30, 48.04, 114.70, 126.88, 128.76, 137.38, 144.81, 145.27, 151.49, 158.82, 70.21. LC-MS (m/z): 309 ([M-H]⁻), 311 ([M+H]⁺). Anal. (C₁₆H₁₈N₆O · 2 HCl · 0.1 H₂O) C, H, N. Purity by HPLC-UV (254 nm)-ESI-MS: 99%.

### Example 15: 3-Amlno-N(9H-purin-6-yl)propionamide hydrochloride.

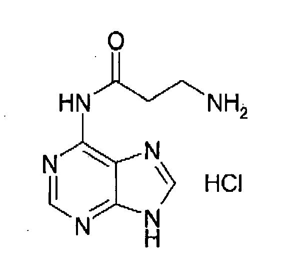

*Reaction conditions:* According to general procedure D (0.16 mmol scale) in 92.7% yield. *Analytical data:* mp. 204 °C. ¹H-NMR (500 MHz, DMSO-d₆) δ 2.97 (t, 2H, *J* = 6.77 Hz), 3.13 (m, 2H), 8.10 (s (br), 3H), 8.59 (s, 1 H), 8.68 (s, 1H), 11.42 (s (br), 1H). ¹³C-NMR (125 MHz, DMSO-d₆) δ 33.25, 34.67, 114.77, 144.62, 145.46, 151.45, 159.98, 170.33. LC-MS (m/z): 205 ([M-H]⁻), 207 ([M+H]⁺). Anal. (C₉H₁₀N₆O · HCl · 2.5 H₂O) C, H, N. Purity by HPLC-UV (254 nm)-ESI-MS: 100%.

### Example. 16: 4-Amino-N(9H-purin-6-yl)butyramide hydrochloride.

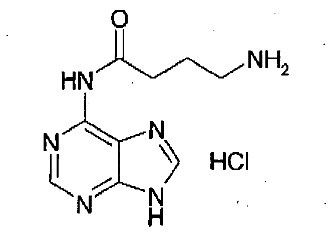

*Reaction conditions:* According to general procedure D (0.08 mmol scale) in 95.9% yield. *Analytical data:* mp 199 °C. ¹H-NMR (500 MHz, DMSO-d₆) δ 1.93 (m, 2H), 2.68 (t, 2H, *J* = 7.25), 2.87 (m, 2H), 8.05 (s (br), 3H), 8.55 (s, 1 H), 8.66 (s, 1 H), 11.29 (s (br), 1 H). ¹³CNMR (125 MHz, DMSO-d₆) δ 22.66, 32.74, 38.38, 114.44, 144.76, 145.42, 151.33, 159.89, 172.44. LC-MS (m/z): 219 ([M-H]⁻), 221 ([M+H]⁺). Anal. (C₉H₁₂N₆O · HCl · 1.75 H₂O) C, H, N. Purity by HPLC-UV (254 nm)-ESI-MS: 99%.

### Biological Assays

### Radioligand binding assay

Competition assays with [8-³H]adenine (27 Ci/mmoi: GE Healthcare) were carried out as described previously (Gorzalka et al., Mol. Pharmacol. 2005, 67, 955-964). In brief, 50µg of rat cortical membrane preparations or 100 µg of protein (HEK293 (human embryonic kidney) cell membrane preparations) was incubated with 10 nM [8-³H]adenine in 50 mM Tris-HCl buffer, pH 7.4, in a total volume of 200 µl. Inhibition curves were determined using 6 to 9 different concentrations of selected adenine derivative. Three separate experiments were performed each in triplicate. Nonspecific binding was determined by using 100 µM adenine. Incubations were carried out for 1 h at room temperature and terminated by rapid filtration through GF/B glass fiber filters (Whatman, Dassel, Germany). Filters were washed three times with ice-cold 50 mM Tris-HCl buffer, pH 7.4, 3 ml each. Filter-bound radioactivity was measured by liquid scintillation counting.

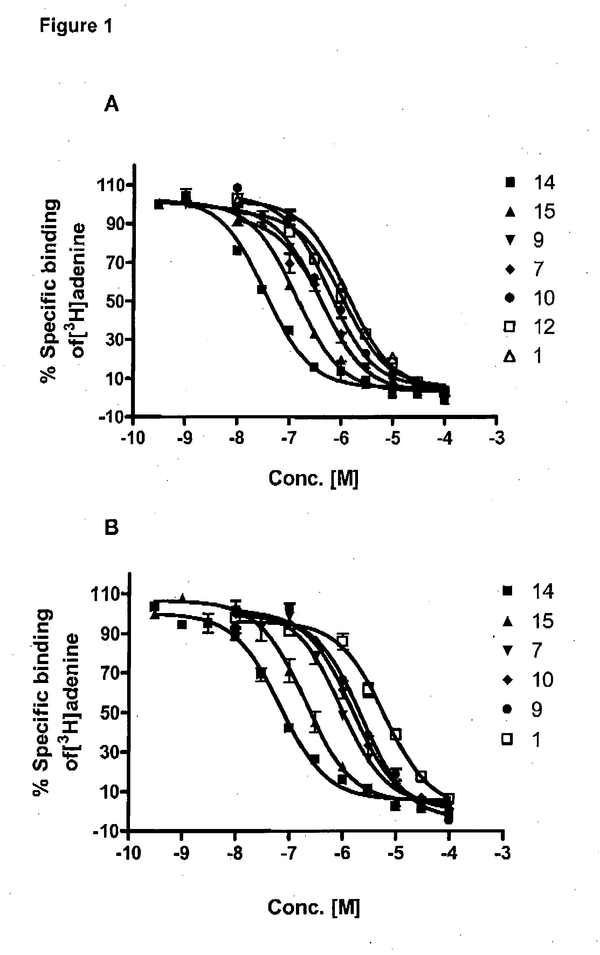

The experimental results are displayed in Figure 1: Competition curves for selected adenine derivatives versus 10 nM [³H]adenine obtained with rat cortical membrane preparation (A) and with a membrane preparations of HEK 293 cells (B).

The quantitative experimental results are summarized in Table 3 here below:

**Table 3: Affinities of selected compounds for human (HEK293 cells) and rat (rat cortex) adenine receptors. The data represent means ± SEM of usually three separate experiments each run in triplicate. (Abbreviations: h = human, r = rat; n.d. = not determined).**

| Entry (ex. no.) | *Structure* | Rat adenine receptor Kᵢ[µM ± SEM] | Human adenine receptor Kᵢ [µM± SEM] vs. [³H]adenine |
|---|---|---|---|
| 1 (2) | 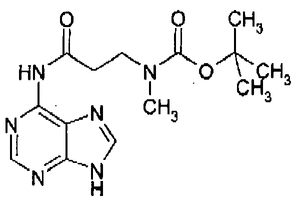 | 0.949 ± 0.135 | 5.47 ± 1.28 |
| 2 (3) | 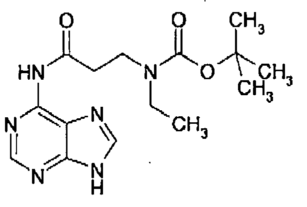 | 2.93 ± 0.40 | 7.70 ± 2.40 |
| 3 (4) | 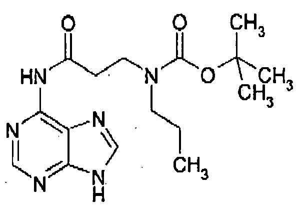 | 11.8 ± 0.8 | ≥ 100 |
| 4 (5) | 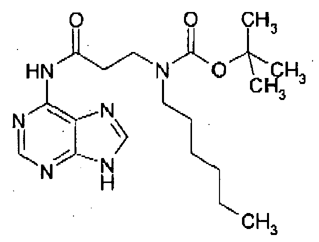 | 1.56 ± 0.026 | 19.7 ± 0.7 |
| 5 (6) | 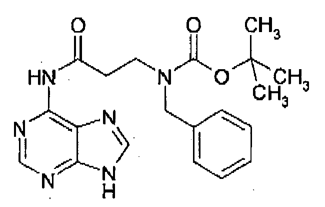 | 9.02 ± 0.55 | 42.1 ± 1.8 |
| 6 (7) | 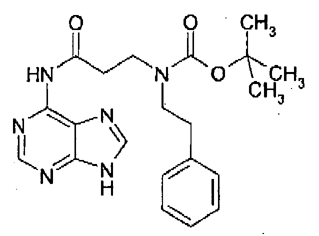 | 9.07 ± 0.80 | ≥ 100 |
| 7 (8) | 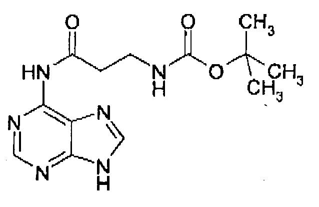 | 0.334 ± 0.031 | 0.719 ± 0.081 |
| 8 (9) | 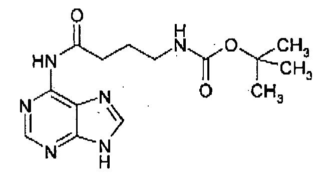 | 3.39 ± 0.56 | 2.68 ± 0.37 |
| 9 (10) | 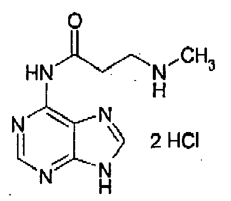 | 0.292 ± 0.056 | 1.83 ± 0.09 |
| 10 (11) | 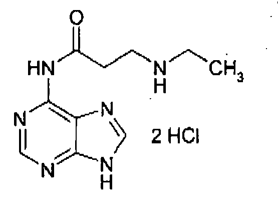 | 0.508 ± 0.163 | 1.34 ± 0.48 |
| 11 (12) | 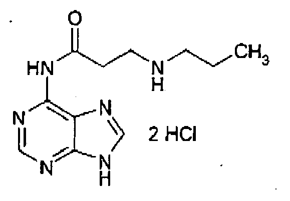 | 1.29 ± 0.24 | 6.85 ± 2.17 |
| 12 (13) | 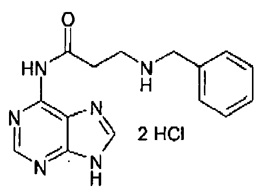 | 0.852 ± 0.131 | 327 ± 1.23 |
| 13 (14) | 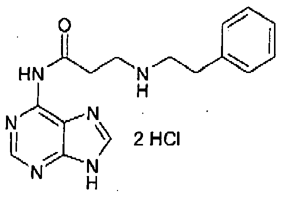 | 2.12 ± 0.07 | 6.93 ± 0.83 |
| 14 (15) | 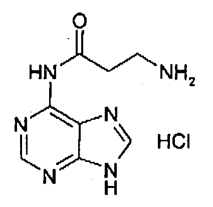 | 0.0215 ± 0.0056 | 0.0447 ± 0.0034 |
| 15 (16) | 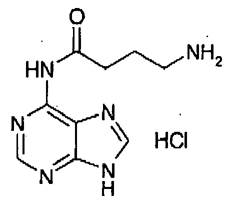 | 0.0805 ± 0.0030 | 0.149 ± 0.045 |

### Functional assays

### Cell culture and DNA transfection

1321N1 cells stably expressing the rat adenine receptor were cultured in DMEM supplemented with 10% (v/v) FCS, L-glutamine (2 mM), penicillin (100 U/ml), streptomycin (100 U/ml) and 400 µg/ml G 418 at 37 °C under a humidified atmosphere containing 5% CO₂. Cells were cultured in 75 cm² flasks (3.2 x 10⁵) for 24 h without antibiotics so that they were 90-95% confluent before transfection. The cAMP responsive reporter plasmid (pCER-luc) was transfected into 1321N1 cells expressing rat adenine receptors using Lipofectamine 2000 as a transfection reagent.

### Luciferase assay

Reporter cells were seeded at a density of 200,000 - 150,000 cells/well in a 24-well plate 18-20 hr prior to assay. After removal of culture medium, cells were washed twice with Hanks' balanced salt solution (containing 20 mM HEPES, pH 7.3). Adenine and selected adenine derivatives were diluted in Hanks' balanced salt solution containing 3 nM isoproterenol and then incubated with reporter cells in the same buffer for 3 h at 37°C. The reaction was stopped by removal of the reaction buffer and the luciferase expression was determined using Bright-Glo^{™} Luciferase assay system (Promega) as described in the kit protocol. Plates were shaken for 2 min to ensure complete lysis of cells. Then the total volume from each well was transferred to a white 96-well plate (Greiner bio-one). Luminescence was determined using a NOVOstar microplate reader (BMG labtech); each well was counted for 2 s at 20 °C.

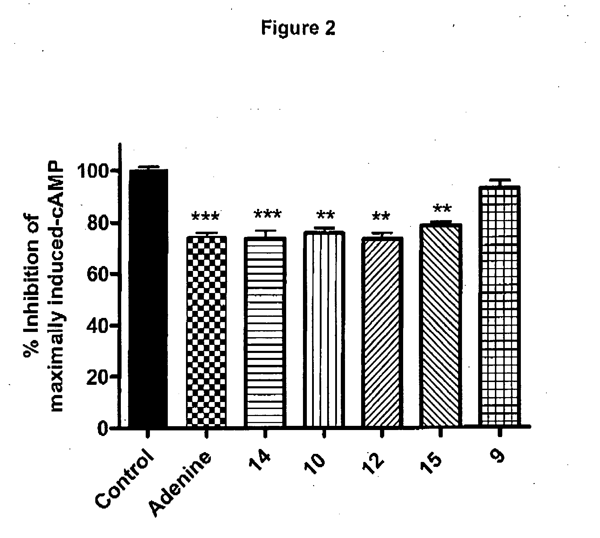

*Figure 2**:* Inhibition of isoproterenol-stimulated cAMP accumulation by selected adenine derivatives at 10 µM in 1321N1 astrocytoma cells stably expressing the mouse adenine receptor. Data are given as percentages of the mean increases in cellular cAMP levels in the presence of isoproterenol alone (control). Adenine and compound 14 were tested at 1 µM (n=3). Compounds 10, 12, 15 and 9 were tested at 10 µM (n=2).

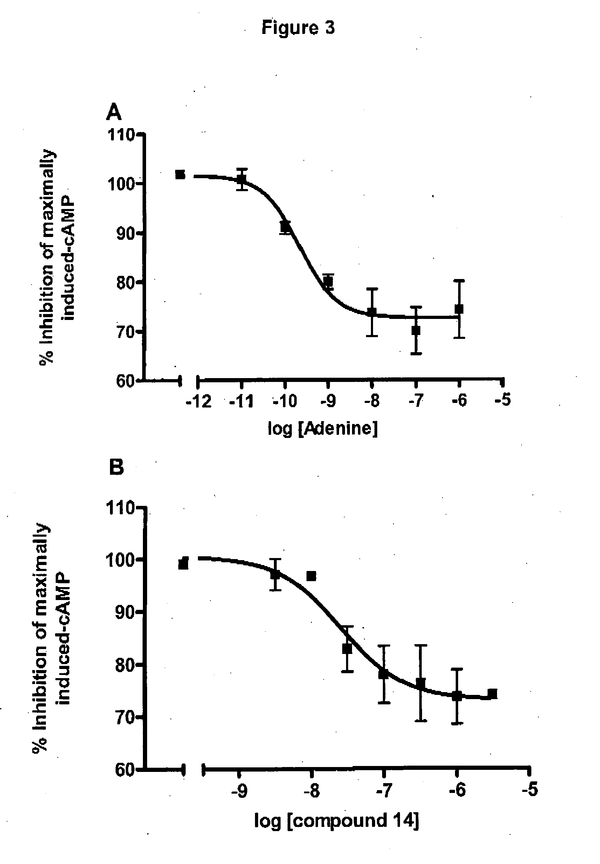

The results are displayed in Figure 3.

## Claims

1. A compound according to general formula (I) 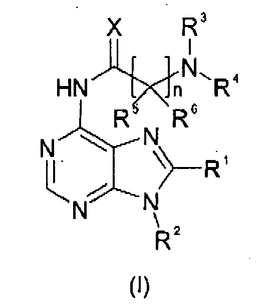 wherein
n is an integer of from 2 to 10;
X is =O, =S, =NH or =NR;
R¹ is -H, -R, -OR, -SR or -NRR';
R², R³, and R⁴ are independently selected from the group consisting of -H; -R; -C(O)R; -C(O)OR; -C(O)NRR', and -NRR';
R⁵ and R⁶ are independently selected from the group consisting of -H; -halogen; -R; an unsubstituted or substituted, unsaturated or saturated 7 to 12-membered bicyclic cycloalkyl ring or heterocycloalkyl ring wherein 1 to 3 ring members are independently selected from N, O and S; an unsubstituted or substituted, unsaturated or saturated 10 to 16-membered tricyclic cycloalkyl ring or heterocycloalkyl ring wherein 1 to 3 ring members are independently selected from N, O and S; -OR; -NRR'; -NO₂; -C(O)R; -C(O)NRR': -S(O)₁₋₂R; -S(O)₁₋₂OR; and -S(O)₁₋₂NRR':
and wherein
R and R' are independently selected from the group consisting of -H; unsubstituted or substituted, unsaturated or saturated, linear or branched -alkyl; unsubstituted or substituted, unsaturated or saturated -cycloalkyl; unsubstituted or substituted, unsaturated or saturated -heterocycloalkyl; unsubstituted or substituted -aryl; unsubstituted or substituted -heteroaryl; unsubstituted or substituted -aryl bonded via unsubstituted or substituted, unsaturated or saturated, linear or branched -alkyl-; unsubstituted or substituted -heteroaryl bonded via unsubstituted or substituted, unsaturated or saturated, linear or branched -alkyl-;
and the physiologically acceptable salts and/or solvates thereof.

2. The compound according to claim 1, wherein
X is =O, =S or =NH;
R², R³, and R⁴ are independently selected from the group consisting of -H; unsubstituted or substituted -C₁-C₁₀ alkyl; unsubstituted or substituted -C₁-C₁₀ alkenyl; unsubstituted or substituted -C₁-C₁₀ alkynyl; unsubstituted or substituted -C₃-C₈ cycloalkyl; unsubstituted or substituted 5- to 10-membered heterocycloalkyl wherein 1 to 3 ring atoms are independently selected from N, O or S; unsubstituted or substituted -C₆-C₁₄ aryl; unsubstituted or substituted 5- to 10membered -heteroaryl wherein 1 to 4 ring atoms are independently selected from N, O or S; unsubstituted or substituted -C₁-C₁₀ alkylC₆-C₁₄ aryl; -C(O)R; -C(O)OR; -C(O)NRR'; and -NRR';
R⁵ and R⁶ are independently selected from the group consisting of -H; -halogen; unsubstituted or substituted -C₁-C₁₀ alkyl; unsubstituted or substituted -C₁-C₁₀ alkenyl; unsubstituted or substituted -C₁-C₁₀ alkynyl; unsubstituted or substituted -C₃-C₈ cycloalkyl; unsubstituted or substituted 5- to 10-membered heterocycloalkyl wherein 1 to 3 ring atoms are independently selected from N, O or S; unsubstituted or substituted -O-C₁-C₁₀ alkyl; unsubstituted or substituted -O-C₃-C₈ cycloalkyl; unsubstituted or substituted C₆-C₁₄ aryl; unsubstituted or substituted 5- to 10-membered -heteroaryl wherein 1 to 4 ring atoms are independently selected from N, O or S; unsubstituted or substituted -C₁-C₁₀ alkyl-C₆-C₁₄ aryl; unsubstituted or substituted -C₁-C₁₀ alkyl-heteroaryl wherein 1 to 4 ring atoms are independently selected from N, O or S; an unsubstituted or substituted, unsaturated or saturated 7 to 12-membered bicyclic cycloalkyl ring or heterocycloalkyl ring wherein 1 to 3 ring members are independently selected from N, O and S; an unsubstituted or substituted, unsaturated or saturated 10 to 16-membered tricyclic cycloalkyl ring or heterocycloalkyl ring wherein 1 to 3 ring members are independently selected from N, O and S; -OR; -NRR'; -NO₂; -C(O)R; -C(O)NRR'; -S(O)₂R; -S(O)₂OR; and -S(O)₂NRR';
and
R and R' are independently selected from the group consisting of -H; unsubstituted or substituted -C₁-C₁₀ alkyl, and -C₁-C₁₀ alkyl-C₆-C₁₄ aryl.

3. The compound according to claim 1 or 2, wherein
X is =O; and/or
R¹ is -H; and/or
R² is -H; and/or
R⁵ is -H; and/or
R⁶ is -H.

4. The compound according to any of the preceding claims, wherein R³ is -H; unsubstituted or substituted -C₁-C₁₀-alkyl; or unsubstituted or substituted -C₁-C₁₀ alkyl-C₆-C₁₄ aryl.

5. The compound according to any of the preceding claims, which is represented by general formula (I-A) or (I-B) 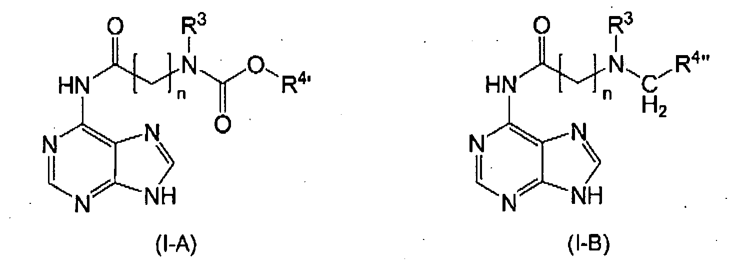 wherein
n and R³ are defined as in any of the preceding claims; and
wherein
R⁴' is unsubstituted or substituted -C₁-C₁₀ alkyl; and
R⁴" is -H; unsubstituted or substituted -C₁-C₉ alkyl; unsubstituted or substituted -C₆-C₁₄ aryl; or unsubstituted or substituted -C₁-C₉ alkyl-C₆-C₁₁ aryl.

6. The compound according to any of the preceding claims, wherein at least one atom is radioactive.

7. The compound according to any of the preceding claims, which is selected from the group consisting of
• 3-(tert-butyloxycarbonyl-methyl-amino)-N(9H-purin-6-yl)propionamide;
• 3-(tert-butyloxycarbonyl-ethyl-amino)-N(9H-purin-6-yl)propionamide;
• 3-(tert-butytoxycarbonyl-propyl-amino)-N(9H-purin-6-yl)propionamide;
• 3-(tert-butyloxycarbonyl-hexyl-amino)-N(9H-purin-6-yl)propionamide;
• 3-(tert-butyloxycarbonyl-benzyl-amino)-N(9H-purin-6-yl)propionamide;
• 3-(tert-butyloxycarbonyl-phenethyl-amino)-N(9H-purin-6-yl)propionamide;
• 3-tert-butyloxycarbonylamino-N(9H-purin-6-yl)propionamide;
• 4-tert-butyloxycarbonylamino-N(9H-purin-6-yl)butyramide;
• 3-methyl-amino-N(9H-purin-6-yl)propionamide;
• 3-ethyl-amino-N(9H-purin-6-yl)propionamide;
• 3-propyl-amino-N(9H-purin-6-yl)propionamide;
• 3-benzyl-amino-N(9H-purin-8-yl)propionamide;
• 3-phonethyl-amino-N(9H-purin-6-yl)propionamide;
• 3-amino-N(9H-purin-6-yl)propionamide; and
• 4-amino-N(9H-purin-6-yl)butyramide;
and the physiologically acceptable salts and/or solvates thereof.

8. A pharmaceutical composition comprising a compound according to any of claims 1 to 7 and a pharmaceutically acceptable carrier.

9. The pharmaceutical composition according to claim 8, which is solid, liquid or pasty.

10. A pharmaceutical dosage form comprising a pharmaceutical composition according to claim 8 or 9.

11. The pharmaceutical dosage form according to claim 10, which is adapted for oral administration.

12. The pharmaceutical dosage form according to claim 10 or 11, which is adapted for administration once daily, twice daily or thrice daily.

13. Use of a compound according to any of claims 1 to 7 as medicament.

14. Use of a compound according to any of claims 1 to 7 for the manufacture of a pharmaceutical composition according to claim 8 or 9 or of a pharmaceutical dosage form according to any of claims 10 to 12 for preventing, ameliorating or treating pain or a neurodegenerative disease.

15. The use according to claim 14, where the neurodegenerative disease is selected from the group consisting of Alzheimer's disease; multiple sclerosis; Huntington's disease; Parkinson's disease; AIDS related dementia; amyotrophic lateral sclerosis; a retinal disease; epilepsy; stroke; acute thromboembolic stroke, focal ischemia, global ischemia, or transient ischemic attack; ischemia resulting from a surgical technique involving prolonged halt of blood flow to the brain; head trauma; spinal trauma; optic nerve stroke; anterior ischemic optic neuropathy; traumatic optic neuropathy; glaucoma; optic neuritis; compressive optic neuropathy; hereditary neuropathy; and schizophrenia.
